(19) Europäisches Patentamt
European Patent Office
Office européen des brevets

(11) EP 1 998 770 B1

(12) FASCICULE DE BREVET EUROPEEN

(45) Date de publication et mention de la délivrance du brevet:
**21.04.2010 Bulletin 2010/16**

(21) Numéro de dépôt: **07731175.1**

(22) Date de dépôt: **22.03.2007**

(51) Int Cl.:
***A61K 31/4375*** *(2006.01)* ***C07D 471/16*** *(2006.01)*
***A61P 31/00*** *(2006.01)*

(86) Numéro de dépôt international:
**PCT/FR2007/000486**

(87) Numéro de publication internationale:
**WO 2007/110500 (04.10.2007 Gazette 2007/40)**

(54) **UTILISATION DE LA CANTHIN-6-ONE ET SES ANALOGUES DANS LE TRAITEMENT DES PATHOLOGIES LIEES AUX MYCOBACTERIES**

VERWENDUNG VON CANTHIN-6-ON UND SEINER ANALOGA BEI DER BEHANDLUNG VON ERKRANKUNGEN DURCH MYCOBAKTERIEN

USE OF CANTHIN-6-ONE AND ITS ANALOGUES IN THE TREATMENT OF PATHOLOGIES LINKED TO MYCOBACTERIA

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **28.03.2006 FR 0602677**

(43) Date de publication de la demande:
**10.12.2008 Bulletin 2008/50**

(73) Titulaire: **Institut de Recherche pour le Développement**
**75480 Paris Cedex 10 (FR)**

(72) Inventeurs:
- **FOURNET, Alain, Robert, François, Maxime**
  **F-40590 OSSAGES (FR)**
- **LAGOUTTE, Delphine**
  **F-92260 Fontenay-Aux-Roses (FR)**
- **POUPON, Erwan**
  **F-92160 ANTONY (FR)**
- **SORIANO-AGATON, Flor**
  **Mexico,D.F. (MX)**

(74) Mandataire: **Grosset-Fournier, Chantal Catherine et al**
**Grosset-Fournier & Demachy**
**54, Rue Saint-Lazare**
**75009 Paris (FR)**

(56) Documents cités:
**EP-A1- 0 043 811 FR-A1- 2 847 474**

- **ODEBIYI O O ET AL: "ANTIMICROBIAL ALKALOIDS FROM A NIGERIAN CHEWING STICK (FAGARA ZANTHOXYLOIDES)" PLANTA MEDICA, THIEME, STUTTGART, DE, vol. 36, no. 3, 1979, pages 204-207, XP008007203 ISSN: 0032-0943**
- **KUO P-C ET AL: "Cytotoxic and Antimalarial [beta]-Carboline Alkaloids from the Roots of Eurycoma longifolia" JOURNAL OF NATURAL PRODUCTS 2003 UNITED STATES, vol. 66, no. 10, 2003, pages 1324-1327, XP002408562 ISSN: 0163-3864**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 998 770 B1

## Description

La présente invention concerne l'utilisation de la canthin-6-one et ses analogues pour la préparation d'un médicament destiné au traitement ou à la prévention de pathologies liées à, ou causées par des mycobactéries, en particulier la tuberculose.

La canthin-6-one est un composé connu qui a été isolé à partir de plantes telles que : *Ailanthus altissima* (Simaroubaceae) par Ohmoto et al., Chem. Pharm. Bull., 1976, 24,1532-1536 ; *Brucea antidysenterica* (Simaroubaceae) par Fukamiya et al., Planta Med., 1987, 53, 140-143 ; *Eurycoma harmandiana* (Simaroubaceae) par Kachanapoom et al., Phytochemistry, 2001, 56, 383-386 ; *Peganum nigellastrum* (Zygophyllaceae) par Ma et al., Phytochemistry, 2000, 53, 1075-1078.

La canthin-6-one et certains de ses dérivés montrent d'intéressantes activités pharmacologiques et notamment une activité antifongique (Thouvenel et al., 2003 ; Soriano-Agatón et al., 2005) et des propriétés trypanocides sur la maladie de Chagas (WO 2004/050092).

Les mycobactéries sont des bactéries très étudiées car responsables de deux graves maladies : la tuberculose et la lèpre.

La tuberculose est une maladie infectieuse, contagieuse et endémique, à tropisme respiratoire très marqué, due à *Mycobacterium tuberculosis* (ou bacille de Koch). *Mycobacterium tuberculosis* appartient au genre des mycobactéries au même titre que le bacille de la lèpre *(Mycobactrium leprae).*

On estime que la tuberculose tue deux millions de personnes chaque année (OMS, 2006). D'autre part, la tuberculose est de plus en plus souvent associée aux infections par le virus VIH (responsable de 13% environ des décès par SIDA dans le monde) ; d'où l'importance d'un traitement anti-tuberculeux chez les immunodéprimés et surtout les Sidéens.

La tuberculose pulmonaire est la plus fréquente, mais il existe des atteintes d'autres localisations (rein, articulation, organes génitaux, péricarde, cerveau...).

La contamination tuberculeuse est essentiellement due à la libération des bacilles par voie aérienne, via des gouttelettes en suspension dans l'air. L'inhalation d'un petit nombre de gouttelettes contaminées suffit à infecter un individu. La transmission par la voie alimentaire (ingestion de viande ou de lait contaminés par *Mycobacterium bovis*) a quasiment disparu.

Le traitement de toutes les formes de tuberculose (tuberculose de l'appareil respiratoire, tuberculose des os et des articulations) repose sur la prise régulière d'antibiotiques. Chez un patient, la primo-infection patente avec signes radiologiques ou généraux doit être considérée comme une tuberculose et traitée comme telle.

Le traitement standard repose sur la prise quotidienne pendant 6 mois d'isoniazide et de rifampicine couplée à l'administration durant les deux premiers mois de pyrazinamide et d'éthambutol (Groupe de travail du conseil supérieur d'hygiène publique en France). Le premier objectif de cette polythérapie est l'action sur les différentes populations de bacilles et la guérison en six mois. Le deuxième objectif est d'empêcher la sélection de mutants résistants à l'origine de rechute à bacilles résistants.

Cependant, à ce jour, le traitement standard ne permet pas toujours de s'affranchir de l'émergence de souches résistantes.

L'un des buts de l'invention est de fournir une nouvelle classe de composés actifs sur le traitement de pathologies associées ou causées par des mycobactéries.

L'un des buis de l'invention est de fournir de nouveaux médicaments contre la tuberculose présentant des propriétés pharmacologiques d'efficacité comparables à celles des médicaments déjà utilisés, mais permettant de remédier à l'émergence de souches résistantes aux médicaments utilisés.

L'un des buts de l'invention est de fournir de nouveaux médicaments contre la tuberculose actifs sur les patients immunodéprimés.

Dans la présente invention, il a été trouvé de façon surprenante que la canthin-6-one et ses dérivés possèdent des propriétés antimycobactériennes.

La présente invention concerne l'utilisation pour la préparation d'un médicament destiné au traitement ou à la prévention de pathologies liées à, ou causées par des mycobactéries, de l'un au moins des composés de formule (I) suivante :

(I)

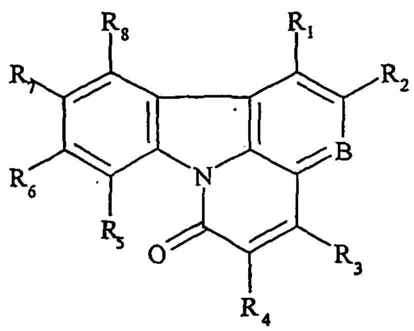

dans laquelle :

- B représente :

  * un atome d'azote, ou
  * un groupe N-oxyde $N^+$-$O^-$, ou
  * un groupe de formule $N^+$-R ou

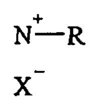

R représentant un groupement alkyle, linéaire, ramifié ou cyclique, saturé ou insaturé, comprenant de 1 à 12 atomes de carbone, de préférence de 1 à 6 atomes de carbone, éventuellement substitué, par exemple par au moins un atome d'halogène, et $X^-$ représente un anion qui peut être choisi parmi les anions minéraux ou organiques.

- $R_1$, $R_2$, $R_3$, $R_4$, $R_5$, $R_6$, $R_7$ et $R_8$ représentent indépendamment l'un de l'autre :

  - un atome d'hydrogène,
  - un groupe alkyle linéaire, ramifié ou cyclique, saturé ou insaturé, comprenant de 1 à 12 atomes de carbone, de préférence de 1 à 6 atomes de carbone,
  - un atome d'halogène choisi parmi le chlore, le fluor, le brome et l'iode,
  - un groupe halogénoalkyle, dans lequel la chaîne alkyle est linéaire, ramifiée ou cyclique, saturée ou insaturée, comprenant de 1 à 12 atomes de carbone, de préférence de 1 à 6 atomes de carbone, et dans lequel le ou les atomes d'halogène sont choisis parmi le fluor, le chlore, le brome et l'iode,
  - une fonction hydroxyle,
  - une fonction nitro -NO,
  - une fonction cyano -CN,
  - une fonction -SH,
  - une fonction acide carboxylique -COOH,
  - une fonction amide $-CONH_2$,
  - une fonction amine $-NH_2$,
  - une fonction alcoxy $-OR_a$, $R_a$ représentant un groupe alkyle, linéaire, ramifié ou cyclique, saturé ou insaturé, comprenant de 1 à 12 atomes de carbone, de préférence de 1 à 6 atomes de carbone,
  - une fonction ester d'alkyle $-COOR_b$, $R_b$ représentant un groupe alkyle, linéaire, ramifié ou cyclique, saturé ou insaturé, comprenant de 1 à 12 atomes de carbone, de préférence de 1 à 6 atomes de carbone,
  - une fonction alkylamide secondaire ($-NHCOR_c$) ou tertiaire ($-N(COR_d)COR_c$), dans laquelle $R_c$ et $R_d$ représentent indépendamment l'un de l'autre un groupe alkyle, linéaire, ramifié ou cyclique, saturé ou insaturé, comprenant de 1 à 12 atomes de carbone, de préférence de 1 à 6 atomes de carbone,
  - une fonction alkylamine secondaire ($-NHR_e$) ou tertiaire ($-NR_eR_f$), dans laquelle $R_e$ et $R_f$ représentent indépendamment l'un de l'autre un groupe alkyle, linéaire, ramifié ou cyclique, saturé ou insaturé, comprenant de 1 à 12 atomes de carbone, de préférence de 1 à 6 atomes de carbone,

- une fonction alkylthio (-$SR_g$), Rg représentant un groupe alkyle, linéaire, ramifié ou cyclique, saturé ou insaturé, comprenant de 1 à 12 atomes de carbone, de préférence de 1 à 6 atomes de carbone,
- un groupement hétérocyclique en $C_2$-$C_6$ comportant 1 à 4 hétéroatomes choisis parmi le soufre, l'azote et l'oxygène,
- un groupement-$SO_2$-$NR_hR_i$ ou un groupement -$NR_h$-$SO_2$-$R_i$, dans lesquels $R_h$ et $R_i$ représentent indépendamment l'un de l'autre un groupe alkyle, linéaire, ramifié ou cyclique, saturé ou insaturé, comprenant de 1 à 12 atomes de carbone, de préférence de 1 à 6 atomes de carbone,

• les groupes $R_1$, $R_2$, $R_3$, $R_4$, $R_5$, $R_6$, $R_7$ et $R_8$ pouvant également former les cyclisations intramoléculaires suivantes :

1/ cyclisation entre $R^1$ et $R^2$ et/ou
2/ cyclisation entre $R^3$ et $R^4$ et/ou
3/ cyclisation entre $R^5$ et $R^6$ et/ou
4/ cyclisation entre $R^6$ et $R^7$ et/ou
5/ cyclisation entre $R^7$ et $R^8$,
6/ cyclisation entre $R^3$ et B, notamment lorsque B représente $N^+$-R,
7/ cyclisation entre $R^2$ et B, notamment lorsque B représente $N^+$-R,

- lesdits cycles ainsi formés étant notamment des cycles aromatiques comprenant de 5 à 30 atomes de carbone, comprenant éventuellement au moins un hétéroatome choisi parmi : O, N, S, les cycles aromatiques étant par exemple choisis parmi le benzène, le naphtalène, la pyridine, le pyrrole, le thiophène, le furane, la pyrazine,
- lesdits cycles étant éventuellement substitués par

  - un atome d'hydrogène,
  - un groupe alkyle linéaire, ramifié ou cyclique, saturé ou insaturé, comprenant de 1 à 12 atomes de carbone, de préférence de 1 à 6 atomes de carbone,
  - un atome d'halogène choisi parmi le chlore, le fluor, le brome et l'iode,
  - un groupe halogénoalkyle, dans lequel la chaîne alkyle est linéaire, ramifiée ou cyclique, saturée ou insaturée, comprenant de 1 à 12 atomes de carbone, de préférence de 1 à 6 atomes de carbone, et dans lequel le ou les atomes d'halogène sont choisis parmi le fluor, le chlore, le brome et l'iode,
  - une fonction hydroxyle,
  - une fonction nitro -NO,
  - une fonction cyano -CN,
  - une fonction -SH,
  - une fonction acide carboxylique -COOH,
  - une fonction amide -$CONH_2$,
  - une fonction amine -$NH_2$,
  - une fonction alcoxy -$OR_a$, $R_a$ représentant un groupe alkyle, linéaire, ramifié ou cyclique, saturé ou insaturé, comprenant de 1 à 12 atomes de carbone, de préférence de 1 à 6 atomes de carbone,
  - une fonction ester d'alkyle -$COOR_b$, $R_b$ représentant un groupe alkyle, linéaire, ramifié ou cyclique, saturé ou insaturé, comprenant de 1 à 12 atomes de carbone, de préférence de 1 à 6 atomes de carbone,
  - une fonction alkylamide secondaire (-$NHCOR_c$) ou tertiaire (-$N(COR_d)COR_c$), dans laquelle $R_c$ et $R_d$ représentent indépendamment l'un de l'autre un groupe alkyl, linéaire, ramifié ou cyclique, saturé ou insaturé, comprenant de 1 à 12 atomes de carbone, de préférence de 1 à 6 atomes de carbone,
  - une fonction alkylamine secondaire (-$NHR_e$) ou tertiaire (-$NR_eR_f$), dans laquelle $R_e$ et $R_f$ représentent indépendamment l'un de l'autre un groupe alkyle, linéaire, ramifié ou cyclique, saturé ou insaturé, comprenant de 1 à 12 atomes de carbone, de préférence de 1 à 6 atomes de carbone,
  - une fonction alkylthio (-$SR_g$), Rg représentant un groupe alkyle, linéaire, ramifié ou cyclique, saturé ou insaturé, comprenant de 1 à 12 atomes de carbone, de préférence de 1 à 6 atomes de carbone,
  - un groupement hétérocyclique en $C_2$-$C_6$ comportant 1 à 4 hétéroatomes choisis parmi le soufre, l'azote et l'oxygène,
  - un groupement-$SO_2$-$NR_hR_i$ ou un groupement -$NR_h$-$SO_2$-$R_i$, dans lesquels $R_h$ et $R_i$ représentent indépendamment l'un de l'autre un groupe alkyle, linéaire, ramifié ou cyclique, saturé ou insaturé, comprenant de 1 à 12 atomes de carbone, de préférence de 1 à 6 atomes de carbone.

La canthin-6-one correspond à la formule (I) dans laquelle les substituants $R_1$ à $R_8$ représentent un atome d'hydrogène et B représente l'azote.

Par mycobactéries, on désigne les espèces du genre *Mycobacterium,* placé dans l'ordre des Actinomycétales

dont le point commun est la mise en évidence par la coloration de Ziehl-Neelsen, de l'acido-alcoolo-résistance (par exemple : Timo Ulrichs et al. J. Pathol. 2005, 205 :633-640 « Modified immunohistological staining allows detection of Ziehl-Neelsen-negativé Mycobacterium tuberculosis organisms and their precise localization in human tissue »).

Selon un mode de réalisation avantageux, les mycobactéries impliquées dans l'invention appartiennent au groupe constitué de *Mycobacterium tuberculosis, Mycobacterium bovis, Mycobacterium smegmatis, Mycobacterium africanum, Mycobacterium leprae, Mycobacterium kansasii, Mycobacterium xenopi, Mycobacterium avium intracellulaire, Mycobacterium scrofulaceum, Mycobacterium marinum, Mycobacterium fortuitum, Mycobacterium chelonei, Mycobacterium ulcerans* et *Mycobacterium abcessus.*

Selon un mode de réalisation avantageux, la présente invention concerne l'utilisation de composés de formule (I) ci-dessus, pour la préparation d'un médicament destiné au traitement ou à la prévention de pathologies liées à, ou causées par *Mycobacterium tuberculosis* ou *Mycobacterium bovis.*

De façon avantageuse, la présente invention concerne l'utilisation d'un composé de formule (I) défini ci-dessus, pour la préparation d'un médicament destiné au traitement ou à la prévention des pathologies suivantes :

- la tuberculose et en particulier
  - la tuberculose de l'appareil respiratoire (tuberculose pulmonaire, des ganglions intra-thoraciques, du larynx, de la trachée, des bronches, la pleurésie tuberculeuse et la primo-infection tuberculeuse de l'appareil respiratoire),
  - la tuberculose du système nerveux (méningite tuberculeuse, polynévrite tuberculeuse, myélite tuberculeuse),
  - la tuberculeuse des os et des articulations,
  - la tuberculose de l'appareil uro-génital,
  - l'adénopathie tuberculeuse périphérique,
  - la tuberculose de l'intestin, du péritoine et des ganglions mésentériques
  - la tuberculose de la peau et du tissu cellulaire sous-cutané
  - la tuberculose de l'oeil et de l'oreille
  - la tuberculose des surrénales
  - la tuberculose miliaire
- l'ulcère de Buruli,
- les infections nosocomiales,
- la lèpre,
- les infections cutanées,
- les infections des tissus mous,
- les ostéomyélites,
- les abcès localisés,
- les pneumonies lipoïdes.

Selon un autre mode de réalisation avantageux, dans les composés de formule (I) dans lesquels B représente

$$N^{+}—R \;;\quad X^{-}$$

$X^-$ est choisi parmi : $Cl^-$, $Br^-$, r, $S^-$, $PO_3^-$ , $NO_3^-$ ; acétate, oxalate, tartrate, succinate, maléate, fumarate, gluconate, citrate, malate, ascorbate, benzoate.

Selon un mode de réalisation avantageux, la présente invention concerne l'utilisation telle que définie ci-dessus, de composés de formule (I) dans laquelle B représente un atome d'azote, les composés répondant à la formule (II) suivante :

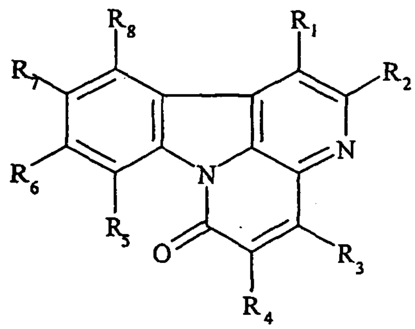

(II)

Selon un mode de réalisation avantageux, la présente invention concerne l'utilisation telle que définie ci-dessus, de composés de formule (I) dans laquelle B représente un groupe N-oxyde NO, les composés répondant à la formule (III) suivante :

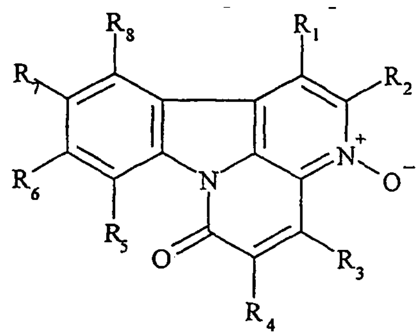

(III)

Selon un mode de réalisation avantageux, la présente invention concerne l'utilisation telle que définie ci-dessus, de composés de formule (I) dans laquelle B représente un groupe de formule,

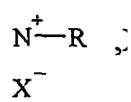

$X^-$ et R étant tels que définis ci-dessus, les composés répondant à la formule (IV) suivante :

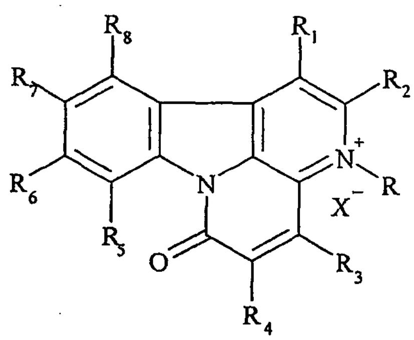

(IV)

R représentant notamment un groupe méthyle, éthyle, par exemple substitué par au moins un atome d'halogène tel que F, Cl, Br ou I.

Selon un mode de réalisation avantageux, la présente invention concerne l'utilisation telle que définie ci-dessus, de composés de formule (I) dans laquelle $R_3$ et $R_4$ forment un cycle benzénique entre eux, les composés répondant à la formule (I-1) suivante :

(I-1) 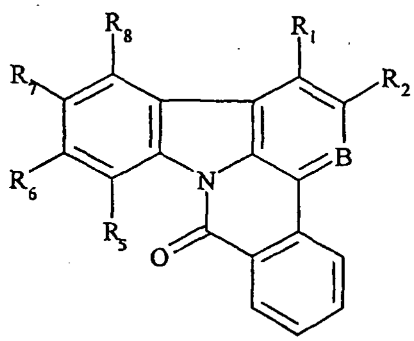

Selon un mode de réalisation avantageux de l'invention, les composés de formule (I) sont choisis parmi :

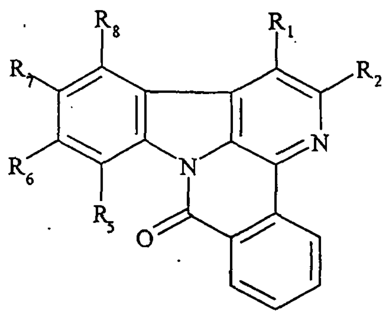

(II-1)

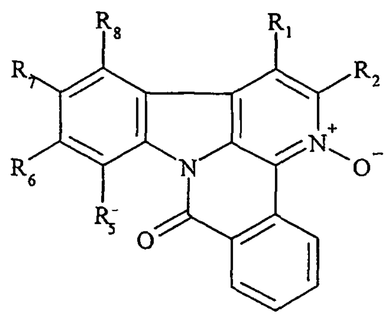

(III-1)

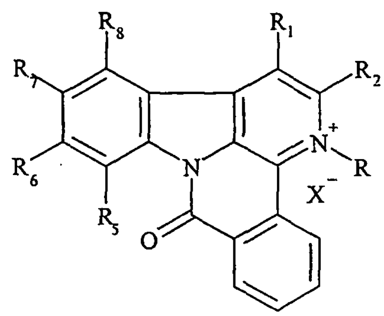

(IV-1)

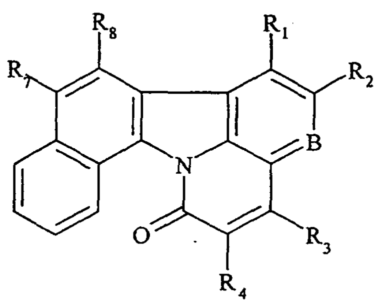

(V-1)

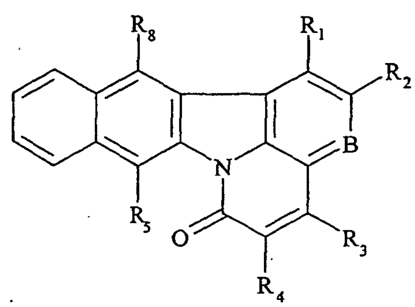

(VI-1)

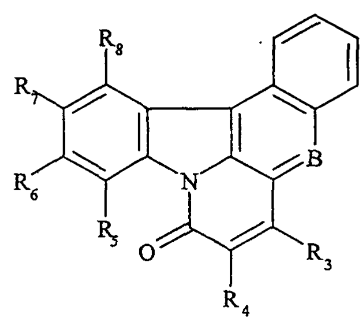

(VII-1)

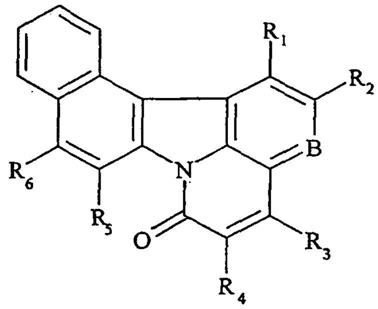

(VIII-1)

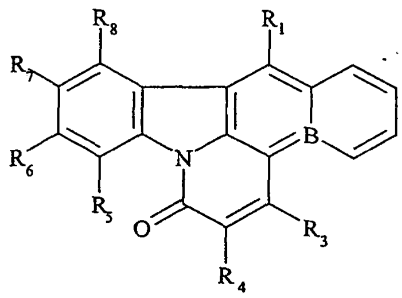

(IX-1)

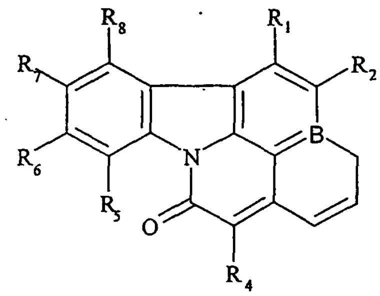

(X-1)

dans lesquelles $R_1$ à $R_8$, B et $X^-$ ont les significations indiquées précédemment.

Selon un autre mode de réalisation avantageux, les composés de formule (I) utilisés sont tels que l'un au moins des radicaux $R_5$ à $R_8$ et notamment $R_6$, représente un atome d'halogène, notamment un atome de fluor.

Selon un autre mode de réalisation avantageux de l'invention, le composé de formule (I) utilisé est la canthin-6-one et répond à la formule suivante :

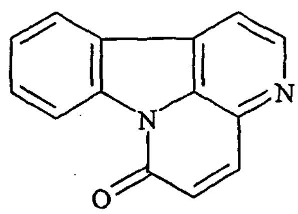

Selon un mode de réalisation avantageux de l'invention, les composés de formule (I) utilisés sont choisis parmi l'un des composés suivants :

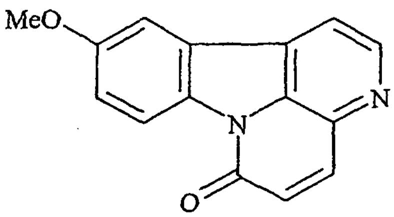

*10-méthoxy-canthin-6-one*

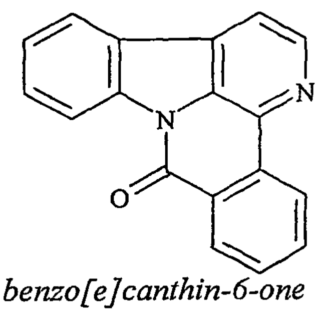

*benzo[e]canthin-6-one*

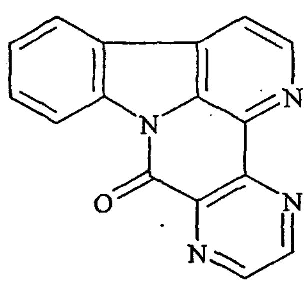

*pyrazine[e]canthin-6-one*

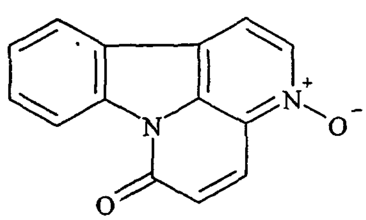

*N-oxyde-canthin-6-one*

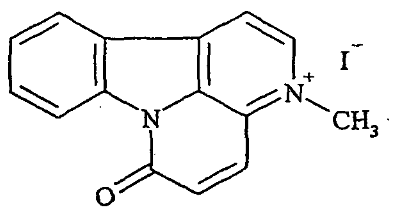

*iodure de N-méthyl-canthin-6-one*

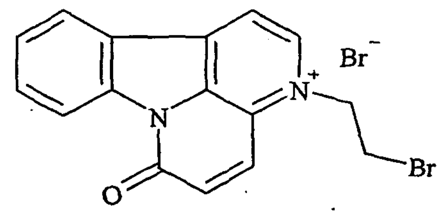

*bromure de N-bromoéthyl-canthin-6-one*

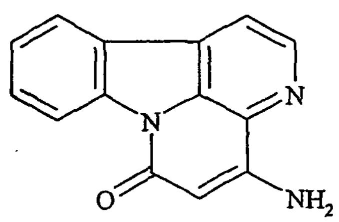

*4-amino-canthin-6-one*

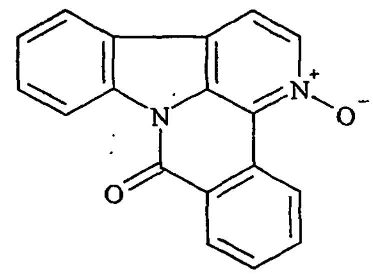

*N-oxyde-benzo[e]canthin-6-one*

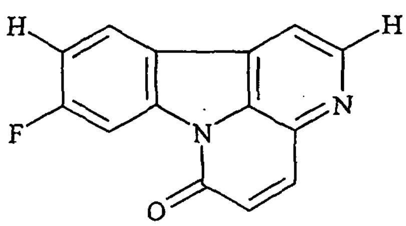

*9-fluoro -canthin-6-one*

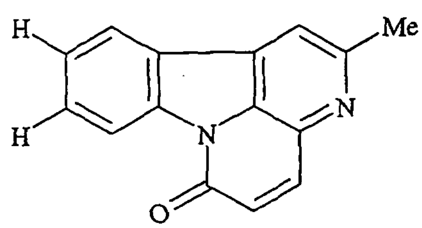

*2-méthyl-canthin-6-one*

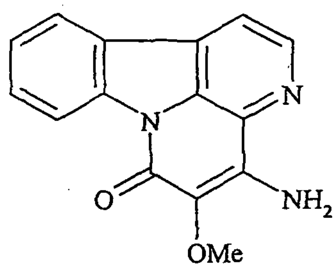

*4-amino-5-méthoxy -canthin-6-one*

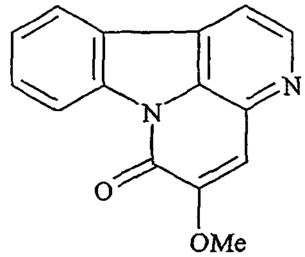

*5-méthoxy-canthin-6-one*

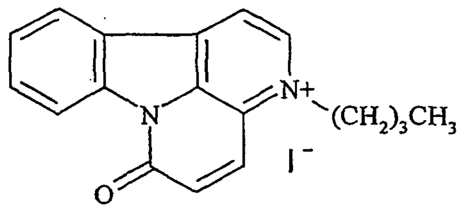

*iodure de $N_3$-butyl-canthin-6-one*

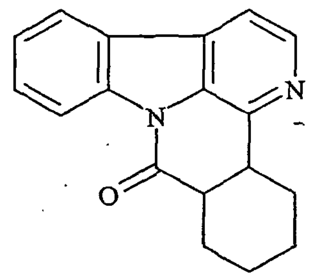

*cyclohexyl[e]-canthin-6-one*

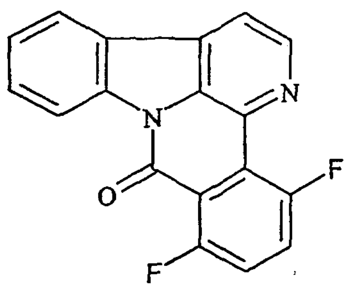

*12,15-difluoro[e]-canthin-6-one*

Les composés de formule (I) définis ci-dessus sont avantageusement administrés à une dose d'environ 0,01 mg/kg/jour à environ 100 mg/kg/jour, de préférence d'environ 0,1 à 50 mg/kg/jour, et avantageusement d'environ 1 à environ 20 mg/kg/jour.

Aucune toxicité n'a été observée dans les différentes expérimentations destinées à déterminer la dose létale (DL50). Les essais ont été réalisés chez la souris en administrant par voie intrapéritonéale, une dose de 200 mg/kg/jour d'un composé de formule (I). Toutes les souris testées ont survécu.

Selon un mode de réalisation avantageux de l'invention, les composés de formule (I) utilisés dans l'invention sont administrés par voie orale.

L'invention concerne aussi le composé, N-oxyde-benzo[e]canthin-6-one, répondant à la formule suivante :

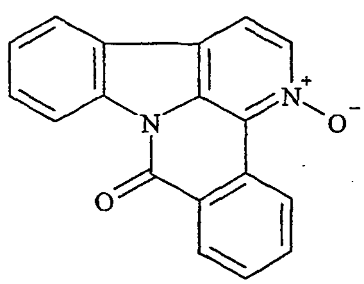

Ce composé est un composé nouveau.

La présente invention a aussi pour objet une composition pharmaceutique comprenant comme substance active le composé N-oxyde-benzo[e]canthin-6-one, répondant à la formule suivante :

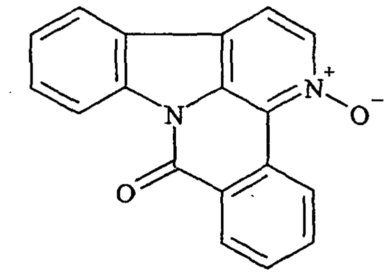

en association avec un véhicule pharmaceutiquement acceptable.

Les composées utilisés dans l'invention peuvent être préparés selon un procédé comme décrit dans la demande de brevet WO 2004/050092. Dans cette demande, la canthin-6-one est isolée d'extraits de plante la contenant et notamment à partir de l'écorce du tronc d'une rutacée identifiée comme *Zanthoxylum chiloperone* var. *angustifolium.*

La canthin-6-one et ses dérivés peuvent être préparés par synthèse chimique totale ou hémi-synthèse comme décrit dans « Soriano-Agatón et al., 2005 ; Journal of Natural Products, volume 68, number 11, novembre 2005 ».

Les exemples suivants servent à illustrer les divers aspects de l'invention plus en détails mais ne doivent pas être interprétés comme des formes limitant l'invention.

EXEMPLE 1

**Synthèse de la canthin-6-one et de ses analogues**

La canthin-6-one et ses analogues sont obtenus par synthèse totale. Il a été développé une voie d'accès rapide au squelette général permettant des pharmacomodulations.

Le schéma suivant présente cette stratégie pour l'obtention de la canthin-6-one :

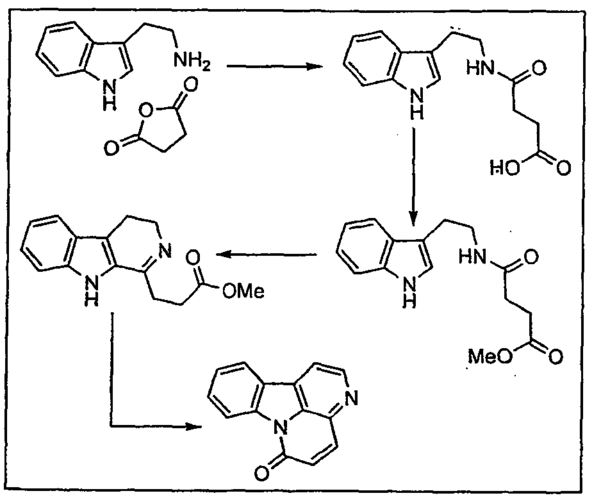

**1- Synthèse de la canthin-6-one à partir de la tryptamine**

■ Acide 4-[2-(1-*H* 3-indolyl)éthylcarbamoyl]-butanoïque

Dans du $CH_2Cl_2$ (50 mL), de la tryptamine (0,01 mole) est dissoute et de l'anhydride succinique (1,1 équiv.) est additionné peu à peu. Cette solution est agitée pendant 18 heures à température ambiante puis concentrée sous pression réduite. Le solide obtenu est trituré avec un minimum de $CH_2Cl_2$, filtré puis séché (>97 %).

■ 4-[2-(1*H*-3-indolyl)éthylcarbamoyl]-butanoate de méthyle

De l'acide 4-[2-(1-*H*-3-indolyl)éthylcarbamoyl]-butanoïque (0,008 mole) est mis en solution dans du méthanol (50 mL). De la résine Amberlyst H-15® (20 % *m*/*m*) est additionnée et le mélange est porté à reflux pendant 18 heures.

Après élimination de la résine par filtration et concentration sous pression réduite, le solide obtenu est trituré dans l'éther éthylique puis filtré et séché (>97 %).

■ 4-(3-4-dihydro-9*H*-β-carboline-1-yl)-butanoate de méthyle

Du 4-[2-(1*H*-3-indolyl)éthylcarbamoyl]-butanoate de méthyle (0,90 mmole) est dissous dans du benzène (10 mL). Du $POCl_3$ (3 équiv.) est additionné goutte à goutte à 5 °C. Le mélange est porté à reflux pendant 1 heure puis concentré sous pression réduite et utilisé tel quel pour l'étape suivante.

■ Canthin-6-one

Dans du $CH_2Cl_2$ (25 mL), du 4-(3-4-dihydro-9*H*-β-carboline-1-yl)-butanoate de méthyle (0,50 mmole) est dissous et du DBU (diazabicycloundécène, 3 équiv.) est additionné. Cette solution est agitée pendant 18 heures à température ambiante puis lavée par une solution aqueuse saturée de $NaHCO_3$. La phase organique est séchée ($Na_2SO_4$) puis concentrée sous pression réduite. La canthin-6-one est purifiée par chromatographie sur colonne de gel de silice (élution : $CH_2Cl_2$/MeOH 99:1, 60 % deux étapes).

**2- Synthèse des dérivés de la canthin-6-one**

Les analogues de la canthin-6-one peuvent être synthétisés en utilisant les techniques comme décrit dans « Soriano-Agatón et al., 2005 ; Journal of Natural Products, volume 68, number 11, novembre 2005 ».

Le composé N-oxyde-benzo[e]canthin-6-one est préparé selon le protocole suivant:

De la benzo[e]canthin-6-one (50 mg - 0,2 mmole) est dissoute dans du $CH_2Cl_2$ (25 mL) puis l'acide m-chloroperbenzoïque (3 équiv.) est ajouté. Cette solution est agitée pendant 18 heures à température ambiante. De l'eau (25 mL) est additionnée et le milieu biphasique agité pendant 1 h. La phase organique est ensuite lavée par une solution aqueuse saturée de $NaHCO_3$ (3 x 20 mL), séchée. ($Na_2SO_4$) puis concentrée sous pression réduite. Le produit obtenu est purifié par chromatographie sur colonne de gel de silice (élution : $CH_2Cl_2$/MeOH 9:1) pour fournir la N-oxyde-benzo [e] canthin-6-one sous forme de poudre (40 mg - 70 %).

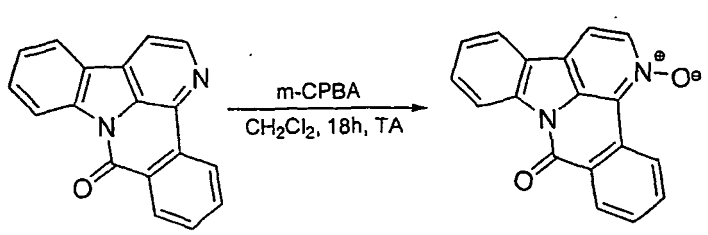


**3- Données analytiques de la canthin-6-one et de ses analogues:**

<u>**Canthin-6-one**</u> :

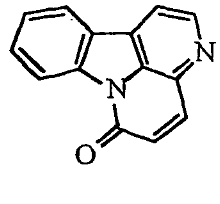

■ poudre jaune microcristalline
■ point de fusion : 161-163 °C
■ IR $\nu_{max}$ $cm^{-1}$: 1673 (-C=O)

■ SM (IE) : *m/z* $[M+Na]^+$ 243
■ SMHR (IC) : pour $C_{14}H_8N_2ONa$ $[M+Na]^+$ 243,0534, trouvé : *m/z* 243,0532
■ $R_f$: 0,6 ($CH_2Cl_2$/MeOH 9:1)

RMN $^1H$ (400 MHz, $CDCl_3$) :

| δ (ppm) | multiplicité | couplage $^nJ$ (Hz) | intégration | attribution |
|---|---|---|---|---|
| 6,87 | d | $^3J$ 9,8 Hz | 1H | H-5 |
| 7,41 | t | $^3J$ 8,0 Hz | 1H | H-10 |
| 7,58 | t | $^3J$ 8,0 Hz | 1H | H-9 |
| 7,80 | d | $^3J$ 5,0 Hz | 1H | H-1 |
| 7,90 | d | $^3J$ 9,8 Hz | 1H | H-4 |
| 7,94 | d | $^3J$ 8,0 Hz | 1H | H-11 |
| 8,50 | d | $^3J$ 8,0 Hz | 1H | H-8 |
| 8,70 | d | $^3J$ 5,0 Hz | 1H | H-2 |

RMN $^{13}C$ (100 MHz, $CDCl_3$) : 116,1 (C-1); 117,0 (C-8); 122,4 (C-11); 124,1 (C-11a) 125,4 (C-10); 128,7 (C-5); 129,9 (C-11b) 130,6 (C-9); 131,7 (C-3b); 135,9 (C-3a); 139,1 (C-7a); 139,3 (C-4); 145,6 (C-2); 159,2 (C-6).

**10-méthoxy-canthin-6-one :**

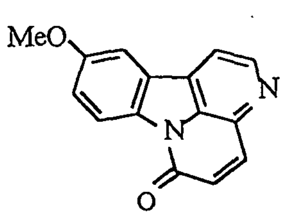

- poudre jaune microcristalline
- point de fusion : 203-205 °C
- IR $\nu_{max}$ $cm^{-1}$ : 1673 (-C=O)
- SM (IC) : *m/z* [M+H]$^+$ 251
- SMHR (IC) : pour $C_{15}H_{11}N_2O_2$ [M+H]$^+$ 251,0821, trouvé : *m/z* 251,0825
- *$R_f$*: 0,6 ($CH_2Cl_2$/MeOH 9:1)

RMN $^1H$ (400 MHz, $CDCl_3$) :

| δ (ppm) | multiplicité | couplage $^nJ$ (Hz) | intégration | attribution |
|---|---|---|---|---|
| 3,96 | s | | 3H | -OMe |
| 6,98 | d | $^3J$ 9,8 Hz | 1H | H-5 |
| 7,27 | dd | $^3J$ 9,0 Hz, $^4J$ 2,1 Hz | 1H | H-9 |
| 7,57 | d | $^4J$ 2,1 Hz | 1H | H-11 |
| 7,96 | d | $^3J$ 5,0 Hz | 1H | H-1 |
| 8,05 | d | $^3J$ 9,8 Hz | 1H | H-4 |
| 8,55 | d | $^3J$ 9,0 Hz | 1H | H-8 |
| 8,81 | d | $^3J$ 5,0 Hz | 1H | H-2 |

RMN $^{13}C$ (100 MHz, $CDCl_3$) : 56,0 (-OMe); 106,7 (C-11); 116,4 (C-1); 118,1 (C-8); 118,3 (C-9); 125,8 (C-11a) 129,4 (C-5); 131,0 (C-11b) 132,5 (C-3b); 133,8 (C-7a); 136,0 (C-3a); 138,7 (C-4); 145,0 (C-2); 158,1 (C-10); 159,2 (C-6).

**Benzo[*e*]canthin-6-one :**

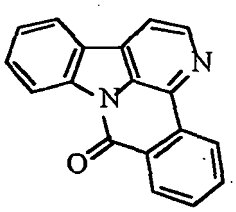

■ poudre blanche microcristalline
■ point de fusion : 228-230 °C
■ IR $\nu_{max}$ $cm^{-1}$ : 1681 (-C=O)
■ SM (IE) : *m/z* $[M+H]^+$ 271
■ SMHR (IC) : pour $C_{18}H_{11}N_2O_2$ $[M+H]^+$ 271,0871, trouvé : *m/z* 271,2073
■ $R_f$: 0,6 ($CH_2Cl_2$/MeOH 9:1)

RMN $^1H$ (400 MHz, $CDCl_3$) :

| δ (ppm) | multiplicité | couplage $^nJ$ (Hz) | intégration | attribution |
|---|---|---|---|---|
| 7,51 | t | $^3J$ 8,0 Hz | 1H | H-10 |
| 7-68-7,76 | m | | 2H | H-14, H-9, |
| 7,87 | t | $^3J$ 8,0 Hz | 1H | H-13 |
| 7,91 | d | $^3J$ 5,0 Hz | 1H | H-1 |
| 8,10 | d | $^3J$ 8,0 Hz | 1H | H-11 |
| 8,61 | d | $^3J$ 8,0 Hz | 1H | H-15 |
| 8,75 | m | | 2H | H-12, H-8 |
| 8,81 | d | $^3J$ 5,0Hz | 1H | H-2 |

RMN $^{13}C$ (100 MHz, $CDCl_3$) : 115,1 (C-1); 117,5 (C-8); 122,4 (C-11); 123,5 (C-12); 124,9 (C-11a); 125,3 (C-10); 129,2 (C-15); 129,5 (C-3a); 130,0 (C-14); 130,4 (C-3b); 130,5 (C-11b); 130,6 (C-9); 133,5 (C-13); 134,7 (C-4); 136,0 (C-5); 139,4 (C-7a); 144,9 (C-2); 159,4 (C-6).

**Pyrazine[*e*]canthin-6-one :**

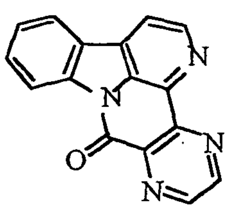

■ poudre brune microcristalline
■ point de fusion : 250-253 °C
■ IR $\nu_{max}$ $cm^{-1}$ : 1699 (-C=O)
■ SM (IE) : *m/z* $[M+Na]^+$ 295
■ SMHR (IC) : pour $C_{16}H_8N_4ONa$ $[M+Na]^+$ 295,0596, trouvé : *m/z* 295,0593
■ $R_f$: 0,4 ($CH_2Cl_2$/MeOH 9:1)

RMN $^1H$ (400 MHz, $CDCl_3$) :

| δ (ppm) | multiplicité | couplage $^nJ$ (Hz) | intégration | attribution |
|---|---|---|---|---|
| 7,58 | t | $^3J$ 8,0 Hz | 1H | H-10 |
| 7,78 | t | $^3J$ 8,0 Hz. | 1H | H-9 |
| 8,09 | d | $^3J$ 5,2 Hz | 1H | H-1 |
| 8,15 | d | $^3J$ 8,0 Hz | 1H | H-11 |

(suite)

| δ (ppm) | multiplicité | couplage $^nJ$ (Hz) | intégration | attribution |
|---|---|---|---|---|
| 8,82 | d | $^3J$ 8,0 Hz | 1H | H-8 |
| 9,02 | d | $^3J$ 5,2 Hz | 1H | H-2 |
| 9,07 | d | $^3J$ 1,6 Hz | 1H | H-13 |
| 9,15 | d | $^3J$ 1,6 Hz | 1H | H-14 |

RMN $^{13}C$ (100 MHz, $CDCl_3$) : 117,3 (C-1); 117,8 (C-8); 122,7 (C-11); 124,7 (C-11a); 126,2 (C-10); 131,4 (C-9); 132,1 (C-11b) 134,1 (C-3b); 139,0 (C-7a); 141,6 (C-3a); 146,0 (C-2); 146,7 (C-13); 147,4 (C-4); 147,4 (C-5); 148,7 (C-14); 157,1 (C-6).

***N*-oxyde-canthin-6-one :**

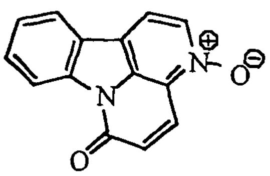

- poudre jaune microcristalline
- point de fusion: 243-245 °C
- IR $\nu_{max}$ $cm^{-1}$ : 1677 (-C=O)
- SM (IC) : *m/z* $[M+H]^+$ 237
- SMHR (IC) : pour $C_{14}H_9N_2O$ $[M+H]^+$ 237,0586, trouvé : *m/z* 237,0584
- $R_f$: 0,6 ($CH_2Cl_2$/MeOH 9:1)

RMN $^1H$ (400 MHz, $CDCl_3$) :

| δ (ppm) | multiplicité | couplage $^nJ$ (Hz) | intégration | attribution |
|---|---|---|---|---|
| 6,92 | d | $^3J$ 10,0 Hz | 1H | H-5 |
| 7,53 | t | $^3J$ 8,0 Hz | 1H | H-10 |
| 7,65 | t | $^3J$ 8,0 Hz | 1H | H-9 |
| 7,82 | d | $^3J$ 6,6 Hz | 1H | H-1 |
| 7,99 | d | $^3J$ 8,0 Hz | 1H | H-11 |
| 8,34 | d | $^3J$ 6,6 Hz | 1H | H-2 |
| 8,38 | d | $^3J$ 10,0 Hz | 1H | H-4 |
| 8,65 | d | $^3J$ 8,0 Hz | 1H | H-8 |

RMN $^{13}C$ (100 MHz, $CDCl_3$) : 117,4 (C-1); 117,7 (C-8); 121,8 (C-11); 123,7 (C-11a); 126,3 (C-5); 128,4 (C-2); 129,1 (C-3b); 129,6 (C-10); 130,0 (C-9); 132,0 (C-11b) 133,0 (C-4); 134,0 (C-3b); 140,0 (C-7-a); 160,1 (C-6). **Iodure de *N*-méthyl- canthin- 6- one :**

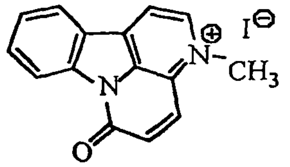

- poudre orange microcristalline
- point de fusion : 238-241 °C
- IR $\nu_{max}$ $cm^{-1}$ : 1684 (-C=O)
- SM (IC) : *m/z* $[M]^+$ 235
- SMHR (IC) : pour $C_{15}H_{11}N_2O$ $[M]^+$ 235,0871, trouvé : *m/z* 235,0873

RMN $^1H$ (400 MHz, $\delta_6$ DMSO) :

| δ (ppm) | multiplicité | couplage $^nJ$ (Hz) | intégration | attribution |
|---|---|---|---|---|
| 4,64 | s | | 3H | -Me |
| 7,41 | d | $^3J$ 10,0 Hz | 1H | H-5 |
| 7,73 | t | $^3J$ 7,8 Hz | 1H | H-10 |
| 7,96 | t | $^3J$ 7,8 Hz | 1H | H-9 |
| 8,57 | m | | 3H | H-8, H-11, H-4 |
| 8,90 | d | $^3J$ 6,3 Hz | 1H | H-1 |
| 9,18 | d | $^3J$ 6,3 Hz | 1H | H-2 |

RMN $^{13}C$ (50 MHz, $\delta_6$ DMSO) : 44,3 (C-Me); 116,8 (C-8); 119,1 (C-1); 122,5 (C-11a); 125,7 (C-11); 127,4 (C-10); 130,2 (C-4); 130,2 (C-3a); 133,3 (C-3b); 133,7 (C-5); 134,7 (C-9); 136,1 (C-11b); 141,4 (C-7a); 142,7 (C-2); 158,0 (C-6).

**Bromure de *N*-bromoéthyl-canthin-6-one :**

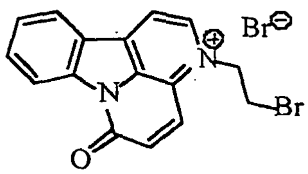

- poudre microcristalline vert pâle
- point de fusion : > 260 °C
- IR $\nu_{max}$ $cm^{-1}$ : 1682 (-C=O)
- SM (IC) : *m/z* $[M]^+$ 248
- SMHR (IC) : pour $C_{16}H_{12}N_2O$ $[M]^+$ 248,0950, trouvé : *m/z* 248,0953

RMN $^1H$ (200 MHz, $D_2O$) :

| δ (ppm) | multiplicité | couplage $^nJ$ (Hz) | intégration | attribution |
|---|---|---|---|---|
| 4,19 | t | $^3J$ 5,8 Hz | 2H | H-2' |
| 5,51 | t | $^3J$ 5,,8 Hz | 2H | H-1' |
| 7,12 | d | $^3J$ 10,2 Hz | 1H | H-5 |
| 7,41 | t | $^3J$ 8,0Hz | 1H | H-10 |
| 7,62 | t | $^3J$ 8,0 Hz | 1H | H-9 |
| 8,06-8,16 | m | | 2H | H-8, H-11 |

(suite)

| δ (ppm) | multiplicité | couplage $^nJ$ (Hz) | intégration | attribution |
|---|---|---|---|---|
| 8,27 | d | $^3J$ 10,2 Hz | 1H | H-4 |
| 8,41 | d | $^3J$ 6,4Hz | 1H | H-1 |
| 8,81 | d | $^3J$ 6,4 Hz | 1H | H-2 |

**N-oxyde-benzo[e]canthin-6-one**

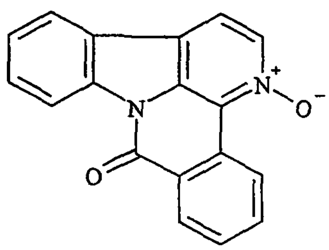

- poudre jaune
- point de fusion: > 250 °C
- IR $\nu_{max}$ $cm^{-1}$ : 1676 (-C=O)
- SM (ES) : *m/z* $[M]^+$ 286
- SMHR (ES) : pour $C_{18}H_{11}N_2O_2$ $[M]^+$ 286,0742, trouvé : *m/z* 286,0741
- $R_f$: 0,6 ($CH_2Cl_2$/MeOH 9 :1)

RMN $^1H$ (200 MHz, $D_2O$) :

| δ (ppm) | multiplicité | couplage $^nJ$ (Hz) | intégration | attribution |
|---|---|---|---|---|
| 7,50 | t | $^3J$ 8,0 Hz | 1H | H-10 |
| 7,72 | t | $^3J$ 8,0 Hz | 1H | H-9 |
| 7,82-7,93 | m | | 2H | H-14, H-13 |
| 8,08 | d | $^3J$ 8,0Hz | 1H | H-11 |
| 8,19 | d | $^3J$ 6,7 Hz | 1H | H-1 |
| 8,47-8,57 | m | | 2H | H-8, H-15 |
| 8,67 | d | $^3J$ 6,7 Hz | 1H | H-2 |
| 9,23 | d | $^3J$ 8,0 Hz | 1H | H-12 |

EXEMPLE 2

**Méthodologie des essais biologiques**

L'activité antimycobactérienne de la canthin-6-one et d'une vingtaine de molécules analogues de la canthin-6-one a été évaluée sur *Mycobacterium bovis* BCG, *Mycobacterium smegmatis, Mycobacterium tuberculosis,* et *Mycobacterium avium.*

*M. bovis* est très proche de *M. tuberculosis* (Institut Pasteur, 2002); une identité de plus de 99 % existe entre les deux génomes.

*M. smegmatis* est une bactérie à croissance rapide (groupe IV de la classification de Runyon), de 3 à 5 μm de longueur, parfois incurvée, ayant tendance à perdre son caractère d'acido-résistance dans les cultures âgées de plus de 5 jours. *M. smegmatis* peut être cultivée dans de la gélose de Middlebrook 7H10 et sur un milieu de Löwenstein-Jensen et est capable de croître sur une gélose de MacConkey dépourvue de cristal violet. Elle assimile le M-inositol, le D-mannitol, le L-rhamnose et le D-sorbitol. *M. smegmatis* est sensible à l'éthambutol (5 μg/mL), et résistante à la rifampicine (25 μg/mL) et à l'isoniazide (10 μg/mL). *M. avium* est une bactérie à croissance lente, en aérobie stricte et non pigmentée. *M. avium* peut être cultivée sur un milieu de Löwenstein-Jensen ou un milieu Coletsos.

Toutes les molécules testées ont été préparées par voie de synthèse chimique.

Les propriétés antimycobactériennes de la canthin-6-one et de ses analogues ont été mises en évidence grâce à l'évaluation de l'activité inhibitrice sur *Mycobacterium bovis* BCG, *Mycobacterium smegmatis, Mycobacterium tuberculosis,* et *Mycobacterium avium*, exprimée en CMI (Concentration Minimale Inhibitrice).

**Essais *in vitro* sur *Mycobacterium bovis* BCG**

Le milieu de culture liquide utilisé est un milieu 7H9 (Middlebrook 7H9 Difco) additionné de tween 80 et d'OADC (Oleic acid, Albumin Fraction V, Dextrose and Catalase) (complément pour milieu de culture Middlebrook OADC).

Les tests sont réalisés sous atmosphère stérile avec des plaques de 96 puits à font plat MICROTEST™. La souche de *Mycobacterium bovis* atténué utilisée est la souche 040812 de l'Institut Pasteur. 180 μL d'une suspension de BCG à $1.10^5$ ufc (unité formant colonie)/mL sont déposés dans chaque cupule (hormis les cupules extérieures qui sont remplies d'eau stérile).

Les molécules à tester sont diluées dans le milieu de culture (pour les molécules très peu solubles les dilutions sont effectuées avec une concentration maximale de 1 % de DMSO (diméthyl sulfoxyde)). 20 μL de chacune des dilutions sont déposés dans les cupules correspondantes. Tous les essais sont répétés trois fois.

Les différents témoins déposés sur chaque plaque sont :

- Témoin positif : BCG seul dans le milieu de culture (200 μL);
- Témoin négatif : milieu de culture seul (200 μL) ;
- Témoin avec solvant : BCG (198 μL) avec DMSO (10 μL).

Les antibiotiques de référence utilisés sont l'isoniazide et l'éthambutol. Les plaques sont ensuite entourées de Parafilm® et mises à l'étuve pendant 6 jours à 37˚C.

Une première lecture des résultats est effectuée à l'aide d'un microscope inversé, au plus faible grossissement. La CMI correspond à la dernière concentration pour laquelle il y a absence d'un voile bactérien.

Les résultats sont confirmés par une coloration par le MTT (bromure de méthylthiazoyltétrazolium). Le MTT est préparé extemporanément dans une solution tamponnée phosphatée à 5 mg/mL et 50 μL de cette solution sont ajoutés dans tous les puits. Puis les plaques sont mises à incuber 4 heures à 37 ˚C à l'abri de la lumière. Au bout de ce laps de temps 50 μL de DMSO/éthanol 50/50 (v/v) sont ajoutés et les plaques sont remises à 37˚C pendant 24 h. Enfin la dernière étape est une lecture de la densité optique (DO) à 570 nm. On pourra déterminer la CMI par l'observation d'une augmentation importante de la DO correspondant à la présence de colonies bactériennes vivantes.

La CMI de l'isoniazide est déterminée par cette méthode :

$$\text{CMI} = 0{,}10\ \mu\text{g/mL}$$

et pour l'éthambutol :

$$\text{CMI} = 5\ \mu\text{g/mL}$$

Tableau

| Activité *in vitro* de la canthin-6-one et analogues sur *Mycobacterium bovis* BCG | | |
|---|---|---|
| MOLECULES | Concentration Minimale Inhibitrice | |
| | CMI(μg/mL) | CMI (μmol/L) |
| 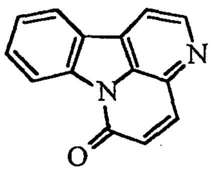 Canthin-6-one | 7,5 | 34,1 |
| 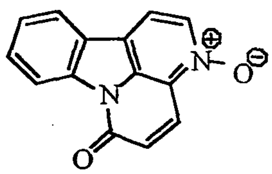 | **2,5<CMI<5*** | **10,6<CMI<21,2*** |
| 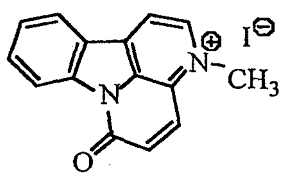 | 7,5 | 31,9 |
| 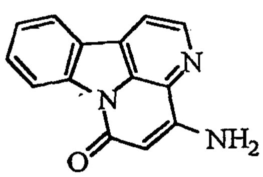 | 10 | 42,5 |
| 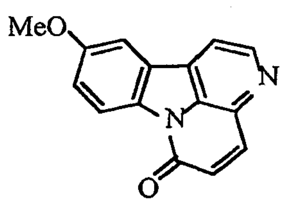 | 7,5 | 30,0 |
| 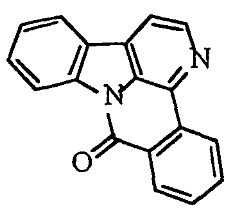 | **2,5<CMI<5*** | **9,2<CMI<18,4*** |
| 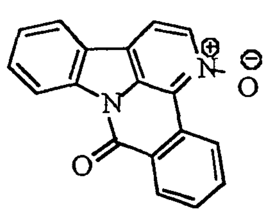 | 10 | 34,8 |

(suite)

| Activité *in vitro* de la canthin-6-one et analogues sur *Mycobacterium bovis* BCG | | |
|---|---|---|
| MOLECULES | Concentration Minimale Inhibitrice | |
| | CMI(µg/mL) | CMI (µmol/L) |
| 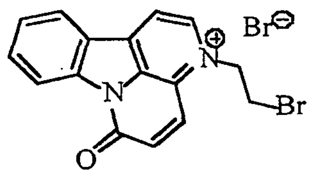 | **1<CMI<5*** | **3,0<CMI<15,3*** |
| 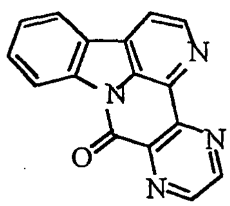 | 10 | 36,8 |
| 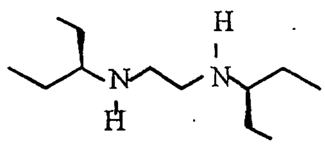 référence : éthambutol | 5 | 24,5 |
| 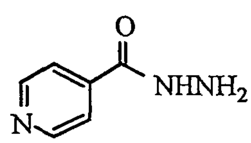 Référence : isionazide | 0,1 | 0,73 |
| • en gras, activité équivalente à celle de l'éthambutol. | | |

Comme on peut le constater dans le tableau de résultats, plusieurs molécules testées ont montré des concentrations minimales inhibitrices (CMI en µg) équivalentes à celles des molécules de référence antituberculeuses, l'éthambutol ou le pyrazinamide, avec des CMI comprises entre 1 et 5 µg/mL.

La canthin-6-one et ses analogues ont montré dans les tests d'activité qui sont exposés ci-dessus, une efficacité étonnante contre *Mycobacterium bovis* BCG.

Les autres composés présentent des propriétés anti-mycobactériennes appréciables.

**Essais *in vitro* sur *Mycobacterium smegmatis***

L'inoculum de mycobactéries 2-3,4 x $10^4$ ufc (unité formant colonie)/mL est préparé à partir de la souche $mc^2155$ de *M. smegmatis* et cultivé dans du milieu Dubos (Difco, USA) additionné de 10 % OADC (Oleic acid, Albumin Fraction V, Dextrose, and Catalase). 100 µl de l'inoculum sont déposés dans chaque puit.

Dans un premier temps, les molécules les plus actives sur *M. bovis* (la canthin-6-one, la N-oxyde-canthin-6-one, la benzo[e]canthin-6-one et le bromure de N-bromoéthyl-canthin-6-one) ont été testées et dans un deuxième temps, tous les analogues de la canthin-6-one sont analysés.

Toutes les molécules à tester sont préparées dans du diméthylsulfoxide (DMSO) à la concentration de 50 mg/ml et mises à congeler jusqu'à leur utilisation.

20 µl de chaque dilution de chaque molécule sont préparés dans 100 µl de milieu de culture Dubos (Difco, USA), puis répartis dans des plaques de 96 puits à des concentrations de 100 à 0,75 µg/ml.

Des puits témoins contenant du DMSO seul et de l'éthambutol (Etibi®, Myambutol®) ou de l'ofloxacine (antibiotique de synthèse appartenant à la famille des fluoroquinolones) sont inclus aux concentrations de 1 μg/l à 1 ng/ml.

Les plaques sont couvertes, scellées et incubées à 37 °C pendant 48 heures.

Les concentrations minimales inhibitrices (CMI) sont déterminées en utilisant la méthode de microdilution de la coloration de la résazurine. Le sel de résazurine en poudre (Sigma) est préparé à la concentration de 0,01 % (poids/vol) dans de l'eau distillée, la solution est ensuite stérilisée par filtration sur membrane de 0.22 μm, puis conservée à 4 °C pendant une semaine.

Dans les plaques incubées pendant 48 heures, 30 μl d'une solution de résazurine sont ajoutés dans chacun des puits. Les plaques sont ensuite incubées à 37°C pendant 24 heures. La variation de la coloration du bleu au rose indique une réduction de la résazurine qui correspond au développement de la colonie bactérienne. La CMI est déterminée comme la concentration la plus faible qui empêche le changement de couleur. La densité optique de chacun des puits est mesurée à 530-630 nm en utilisant un lecteur de micro-plaques. La CMI est déterminée en traçant la courbe-réponse dose-densité optique.

La CMI de l'ofloxacine est déterminée par cette méthode :

$$CMI = 1\ \mu g/ml,$$

et pour l'éthambutol :

$$CMI = 0{,}5\ \mu g/ml.$$

Plusieurs molécules ont montré une efficacité équivalente à celle des molécules de référence antituberculeuses.

La canthin-6-one et ses analogues se sont montrés, dans les tests d'activité qui sont exposés ci-dessus, efficaces contre *Mycobacterium smegmatis.*

**Essais *in vitro* sur *Mycobacterium tuberculosis***

Les mycobactéries de croissance lente comme *M. tuberculosis* H37Rv sont cultivées dans du milieu Löwenstein-Jensen (Difco Laboratories, USA).

Dans un premier temps, les molécules les plus actives sur *M. bovis* et citées ci-dessus ont été testées et dans un deuxième temps, tous les analogues de la canthin-6-one sont analysés.

Les molécules à tester sont diluées dans un milieu de culture, pour les molécules très peu solubles, les dilutions sont effectuées avec une concentration maximale de 1 % de DMSO (diméthylsulfoxyde). Tous les essais sont répétés trois fois.

Les concentrations minimales inhibitrices sont déterminées dans un milieu agar 7H11 additionné de 10 % d'OADC (Oleic acid, Albumin Fraction V, Dextrose and Catalase) contenant $10^3$ à $10^5$ ufc (unité formant colonie)/ml.

Les colonies de mycobactéries sont énumérées après 21 à 30 jours d'incubation à 37°C. La concentration minimale inhibitrice est déterminée comme la concentration la plus faible de dérivés de canthin-6-one qui réduit la croissance de 1% au moins en la comparant à une colonie témoin non traitée.

La CMI de la rifampicine (antibiotique antituberculeux de la famille des rifamycines) est :

$$CMI = 0{,}1\ \mu g/ml$$

et pour l'ofloxacine :

$$CMI = 1\ \mu g/ml.$$

Plusieurs molécules ont montré une efficacité équivalente à celle des molécules de référence antituberculeuses.

La canthin-6-one et ses analogues se sont montrés, dans les tests d'activité qui sont exposés ci-dessus, efficaces contre *Mycobacterium tuberculosis.*

**Essais *in vitro* sur *Mycobacterium avium***

Les mycobactéries de croissance lente comme *M. avium* 101 sont cultivées dans du milieu Löwenstein-Jensen (Difco Laboratories, USA).

Dans un premier temps, les molécules les plus actives sur *M. bovis* et citées ci-dessus ont été testées et dans un deuxième temps, tous les analogues de la canthin-6-one sont analysés.

Les molécules à tester sont diluées dans un milieu de culture, pour les molécules très peu solubles, les dilutions sont effectuées avec une concentration maximale de 1 % de DMSO (diméthylsulfoxyde). Tous les essais sont répétés trois fois.

Les concentrations minimales inhibitrices sont déterminées dans un milieu agar 7H11 additionné de 10 % d'OADC (Oleic acid, Albumin Fraction V, Dextrose and Catalase) contenant $10^3$ à $10^5$ ufc (unité formant colonie)/ml.

Les colonies de mycobactéries sont énumérées après 21 à 30 jours d'incubation à 37˚C. La concentration minimale inhibitrice est déterminée comme la concentration la plus faible de dérivés de canthin-6-one qui réduit la croissance de 1% au moins en la comparant à une colonie témoin non traitée.

La CMI de l'isoniazide (antituberculeux dérivé de l'acide isonicotinique) est :

$$CMI = 0{,}1\ \mu g/ml$$

et pour l'ofloxacine :

$$CMI = 6{,}2\ \mu g/ml.$$

Plusieurs molécules ont montré une efficacité équivalente à celle des molécules de référence antituberculeuses.

La canthin-6-one et ses analogues se sont montrés, dans les tests d'activité qui sont exposés ci-dessus, efficaces contre *Mycobacterium avium.*

EXEMPLE 3

**Essais *in vivo* sur *Mycobacterium bovis* BCG**

Animaux d'expérimentation :

Les animaux utilisés sont des souris femelles C57Bl/6.

Infection :

200 μL d'une suspension de BCG à la concentration de $5 \times 10^5$ UV/mL (UV : unités viables) dans du sérum physiologique sont inoculés aux différentes souris après 7 jours de stabulation.

Traitement :

Le traitement commence 15 jours après inoculation du BCG et dure 5 jours (1 fois par jour). Toutes les molécules testées (la canthin-6-one et ses analogues) sont mises en suspension dans du sérum physiologique (avec 1 % de DMSO) et 200 μL sont administrés aux souris par voie intrapéritonéale.

Le produit de référence utilisé est l'isoniazide à la dose de 25 mg/kg/j et les molécules sont testées à la dose de 20 mg/kg/j.

Paramètres étudiés :

L'activité antituberculeuse est évaluée par le comptage du nombre de bactéries au niveau de la rate, du foie et des poumons.

Ces organes sont prélevés à la fin du traitement après sacrifice des souris. Les organes sont par la suite broyés dans du tampon Sauton et déposés sur des géloses 7H11 additionné d'OADC (Oleic acid, Albumin Fraction V, Dextrose et catalase).

Le comptage de chaque boite se fait 2 à 3 semaines après une incubation à 37 °C.
Plusieurs molécules ont montré une efficacité équivalente à celle de l'isoniazide.
La canthin-6-one et ses analogues se sont montrés, dans les tests d'activité *in vivo* qui sont exposés ci-dessus, efficaces contre *Mycobacterium bovis* BCG.

## Revendications

1. Utilisation pour la préparation d'un médicament destiné au traitement ou à la prévention de pathologies liées à, ou causées par des mycobactéries, de l'un au moins des composés de formule (I) suivante :

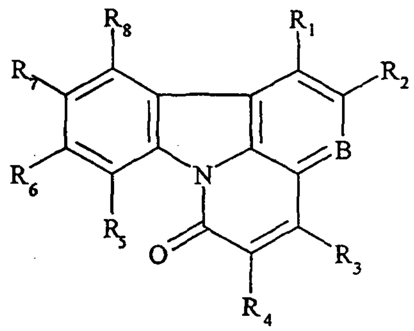

(I)

dans laquelle :

- B représente :

  * un atome d'azote, ou
  * un groupe N-oxyde $N^+$-$O^-$, ou
  * un groupe de formule $N^+$-R ou

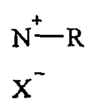

R représentant un groupement alkyle, linéaire, ramifié ou cyclique, saturé ou insaturé, comprenant de 1 à 12 atomes de carbone, de préférence de 1 à 6 atomes de carbone, éventuellement substitué, par exemple par au moins un atome d'halogène, et $X^-$ représente un anion qui peut être choisi parmi les anions minéraux ou organiques.

- $R_1$, $R_2$, $R_3$, $R_4$, $R_5$, $R_6$, $R_7$ et $R_8$ représentent indépendamment l'un de l'autre :

  - un atome d'hydrogène,
  - un groupe alkyle linéaire, ramifié ou cyclique, saturé ou insaturé, comprenant de 1 à 12 atomes de carbone, de préférence de 1 à 6 atomes de carbone,
  - un atome d'halogène choisi parmi le chlore, le fluor, le brome et l'iode,
  - un groupe halogénoalkyle, dans lequel la chaîne alkyle est linéaire, ramifiée ou cyclique, saturée ou insaturée, comprenant de 1 à 12 atomes de carbone, de préférence de 1 à 6 atomes de carbone, et dans lequel le ou les atomes d'halogène sont choisis parmi le fluor, le chlore, le brome et l'iode,
  - une fonction hydroxyle,
  - une fonction nitro -NO,
  - une fonction cyano -CN,
  - une fonction -SH,
  - une fonction acide carboxylique -COOH,
  - une fonction amide $-CONH_2$,
  - une fonction amine $-NH_2$,

- une fonction alcoxy -$OR_a$, $R_a$ représentant un groupe alkyl, linéaire, ramifié ou cyclique, saturé ou insaturé, comprenant de 1 à 12 atomes de carbone, de préférence de 1 à 6 atomes de carbone,
- une fonction ester d'alkyle -$COOR_b$, $R_b$ représentant un groupe alkyle, linéaire, ramifié ou cyclique, saturé ou insaturé, comprenant de 1 à 12 atomes de carbone, de préférence de 1 à 6 atomes de carbone,
- une fonction alkylamide secondaire (-$NHCOR_c$) ou tertiaire (-$N(COR_d)COR_c$), dans laquelle $R_c$ et $F_d$ représentent indépendamment l'un de l'autre un groupe alkyle, linéaire, ramifié ou cyclique, saturé ou insaturé, comprenant de 1 à 12 atomes de carbone, de préférence de 1 à 6 atomes de carbone,
- une fonction alkylamine secondaire (-$NHR_e$) ou tertiaire (-$NR_eR_f$), dans laquelle $R_e$ et $R_f$ représentent indépendamment l'un de l'autre un groupe alkyle, linéaire, ramifié ou cyclique, saturé ou insaturé, comprenant de 1 à 12 atomes de carbone, de préférence de 1 à 6 atomes de carbone,
- une fonction alkylthio (-$SR_g$), $R_g$ représentant un groupe alkyle; linéaire, ramifié ou cyclique, saturé ou insaturé, comprenant de 1 à 12 atomes de carbone, de préférence de 1 à 6 atomes de carbone,
- un groupement hétérocyclique en $C_2$-$C_6$ comportant 1 à 4 hétéroatomes choisis parmi le soufre, l'azote et l'oxygène,
- un groupement-$SO_2$-$NR_hR_i$ ou un groupement -$NR_h$-$SO_2$-$R_i$, dans lesquels $R_h$ et $R_i$ représentent indépendamment l'un de l'autre un groupe alkyle, linéaire, ramifié ou cyclique, saturé ou insaturé, comprenant de 1 à 12 atomes de carbone, de préférence de 1 à 6 atomes de carbone,

• les groupes $R_1$, $R_2$, $R_3$, $R_4$, $R_5$, $R_6$, $R_7$ et $R_8$ pouvant également former les cyclisations intramoléculaires suivantes :

1/ cyclisation entre $R^1$ et $R^2$ et/ou
2/ cyclisation entre $R^3$ et $R^4$ et/ou
3/ cyclisation entre $R^5$ et $R^6$ et/ou
4/ cyclisation entre $R^6$ et $R^7$ et/ou
5/ cyclisation entre $R^7$ et $R^8$,
6/ cyclisation entre $R^3$ et B, notamment lorsque B représente $N^+$-R,
7/ cyclisation entre $R^2$ et B, notamment lorsque B représente $N^+$-R,
- lesdits cycles ainsi formés étant notamment des cycles aromatiques comprenant de 5 à 30 atomes de carbone, comprenant éventuellement au moins un hétéroatome choisi parmi : O, N, S, les cycles aromatiques étant par exemple choisis parmi le benzène, le naphtalène, la pyridine, le pyrrole, le thiophène, le furane, la pyrazine,
- lesdits cycles étant éventuellement substitués par

  - un atome d'hydrogène,
  - un groupe alkyle linéaire, ramifié ou cyclique, saturé ou insaturé, comprenant de 1 à 12 atomes de carbone, de préférence de 1 à 6 atomes de carbone,
  - un atome d'halogène choisi parmi le chlore, le fluor, le brome et l'iode,
  - un groupe halogénoalkyle, dans lequel la chaîne alkyle est linéaire, ramifiée ou cyclique, saturée ou insaturée, comprenant de 1 à 12 atomes de carbone, de préférence de 1 à 6 atomes de carbone, et dans lequel le ou les atomes d'halogène sont choisis parmi le fluor, le chlore, le brome et l'iode,
  - une fonction hydroxyle,
  - une fonction nitro -NO,
  - une fonction cyano -CN,
  - une fonction -SH,
  - une fonction acide carboxylique -COOH,
  - une fonction amide -$CONH_2$,
  - une fonction amine -$NH_2$,
  - une fonction alcoxy -$OR_a$, $R_a$ représentant un groupe alkyle, linéaire, ramifié ou cyclique, saturé ou insaturé, comprenant de 1 à 12 atomes de carbone, de préférence de 1 à 6 atomes de carbone,
  - une fonction ester d'alkyle -$COOR_b$, $R_b$ représentant un groupe alkyle, linéaire, ramifié ou cyclique, saturé ou insaturé, comprenant de 1 à 12 atomes de carbone, de préférence de 1 à 6 atomes de carbone,
  - une fonction alkylamide secondaire (-$NHCOR_c$) ou tertiaire (-$N(COR_d)COR_c$), dans laquelle $R_c$ et $R_d$ représentent indépendamment l'un de l'autre un groupe alkyle, linéaire, ramifié ou cyclique, saturé ou insaturé, comprenant de 1 à 12 atomes de carbone, de préférence de 1 à 6 atomes de carbone,
  - une fonction alkylamine secondaire (-$NHR_e$) ou tertiaire (-$NR_eR_f$), dans laquelle $R_e$ et $R_f$ représentent indépendamment l'un de l'autre un groupe alkyle, linéaire, ramifié ou cyclique, saturé ou insaturé, comprenant de 1 à 12 atomes de carbone, de préférence de 1 à 6 atomes de carbone,

- une fonction alkylthio ($-SR_g$), $R_g$ représentant un groupe alkyle, linéaire, ramifié ou cyclique, saturé ou insaturé, comprenant de 1 à 12 atomes de carbone, de préférence de 1 à 6 atomes de carbone,
- un groupement hétérocyclique en $C_2$-$C_6$ comportant 1 à 4 hétéroatomes choisis parmi le soufre, l'azote et l'oxygène,
- un groupement-$SO_2$-$NR_hR_i$ ou un groupement -$NR_h$-$SO_2$-$R_i$, dans lesquels $R_h$ et $R_i$ représentent indépendamment l'un de l'autre un groupe alkyle, linéaire, ramifié ou cyclique, saturé ou insaturé, comprenant de 1 à 12 atomes de carbone, de préférence de 1 à 6 atomes de carbone.

**2.** Utilisation selon la revendication 1, **caractérisée en ce que** les mycobactéries sont choisies dans le groupe constitué de *Mycobacterium tuberculosis, Mycobacterium bovis, Mycobacterium smegmatis, Mycobacterium africanum, Mycobacterium leprae, Mycobacterium kansasii, Mycobacterium xenopi, Mycobacterium avium intracellulaire, Mycobacterium scrofulaceum, Mycobacterium marinum, Mycobacterium fortuitum, Mycobacterium chelonei, Mycobacterium ulcerans et Mycobacterium abcessus,* notamment *Mycobacterium tuberculosis* et *Mycobacterium bovis.*

**3.** Utilisation selon la revendication 1, **caractérisée en ce que** les pathologies liées à, ou causées par des mycobactéries, sont choisies parmi :

- la tuberculose et en particulier

  - la tuberculose de l'appareil respiratoire (tuberculose pulmonaire, des ganglions intra-thoraciques, du larynx, de la trachée, des bronches, la pleurésie tuberculeuse et la primo-infection tuberculeuse de l'appareil respiratoire),
  - la tuberculose du système nerveux (méningite tuberculeuse, polynévrite tuberculeuse, myélite tuberculeuse),
  - la tuberculeuse des os et des articulations,
  - la tuberculose de l'appareil uro-génital,
  - l'adénopathie tuberculeuse périphérique,
  - la tuberculose de l'intestin, du péritoine et des ganglions mésentériques
  - la tuberculose de la peau et du tissu cellulaire sous-cutané
  - la tuberculose de l'oeil et de l'oreille
  - la tuberculose des surrénales
  - la tuberculose miliaire

- l'ulcère de Buruli,
- les infections nosocomiales,
- la lèpre,
- les infections cutanées,
- les infections des tissus mous,
- les ostéomyélites,
- les abcès localisés,
- les pneumonies lipoïdes.

**4.** Utilisation selon l'une quelconque des revendications 1 à 3, **caractérisée en ce que** $X^-$ est choisi parmi : $Cl^-$, $Br^-$, $T^-$, $S^-$, $PO_3^-$, $NO_3^-$, acétate, oxalate, tartrate, succinate, maléate, fumarate, gluconate, citrate, malate, ascorbate, benzoate.

**5.** Utilisation selon l'une quelconque des revendications 1 à 4, **caractérisée en ce que** B représente un atome d'azote, les composés répondant à la formule (II) suivante :

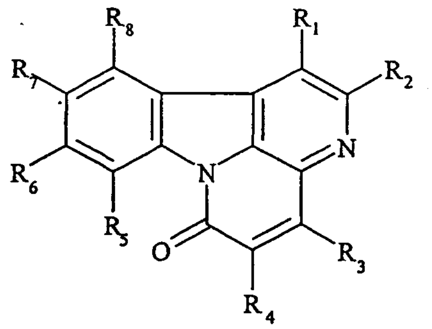

(II)

6. Utilisation selon l'une quelconque des revendications 1 à 4, **caractérisée en ce que** B représente un groupe N-oxyde NO, les composés répondant à la formule (III) suivante :

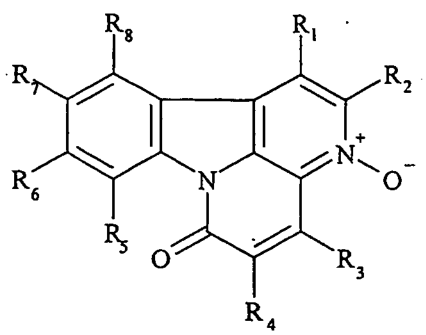

(III)

7. Utilisation selon l'une quelconque des revendications 1 à 4, **caractérisée en ce que** B représente un groupe de formule

$$\overset{+}{N}-R\ ,\quad X^{-}$$

$X^-$ et R étant tels que définis dans la revendication 1, les composés répondant à la formule (IV) suivante :

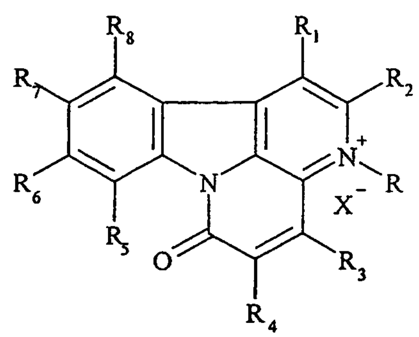

(IV)

R représentant notamment un groupe méthyle, éthyle, par exemple substitué par au moins un atome d'halogène tel que F, Cl, Br ou I.

8. Utilisation selon l'une quelconque des revendications 1 à 4, **caractérisée en ce que** $R_3$ et $R_4$ forment un cycle benzénique entre eux, les composés répondant à la formule (I-1) suivante :

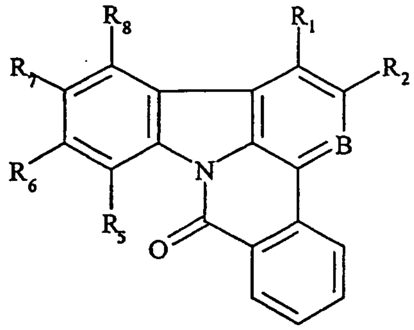

(I-1)

**9.** Utilisation selon l'une quelconque des revendications 1 à 4, **caractérisée en ce que** les composés sont choisis parmi:

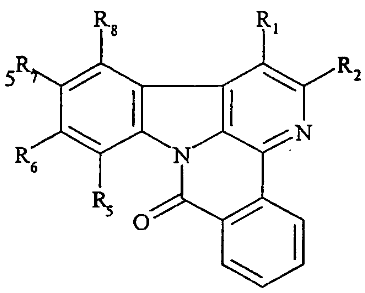

(II-1)

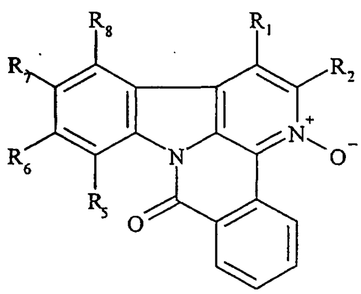

(III-1)

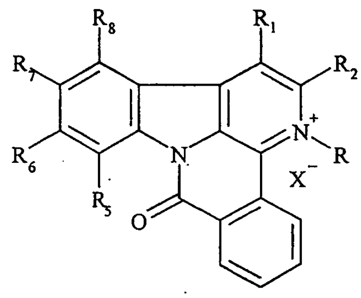

(IV-1)

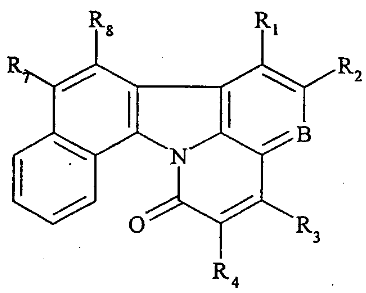

(V-1)

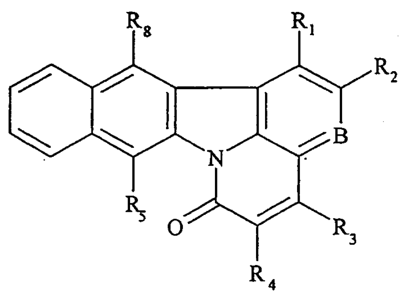

(VI-1)

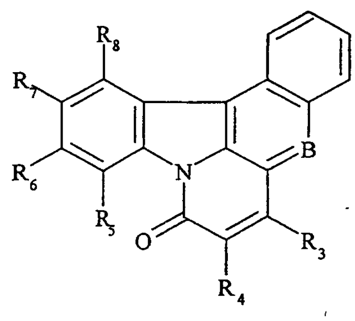

(VII-1)

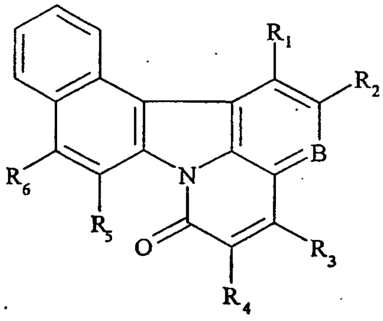

(VIII-1)

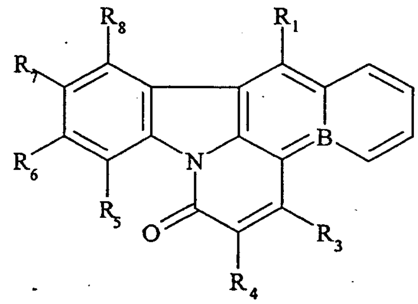

(IX-1)

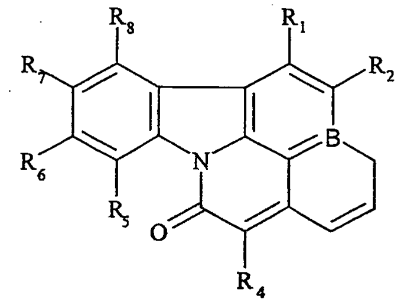

(X-1)

dans lesquelles $R_1$ à $R_8$, B et $X^-$ ont les significations indiquées aux revendications 1 à 8.

**10.** Utilisation selon l'une quelconque des revendications 1 à 9, **caractérisée en ce que** l'un au moins des radicaux $R_5$ à $R_8$ et notamment $R_6$ représente un atome d'halogène, notamment un atome de fluor.

**11.** Utilisation selon la revendication 1 à 4, **caractérisée en ce que** B représente un atome d'azote et **en ce que** $R_1$, $R_2$, $R_3$, $R_4$, $R_5$, $R_6$, $R_7$ et $R_8$ représentent un atome d'hydrogène, ledit composé répondant à la formule suivante :

*(canthin-6-one)*

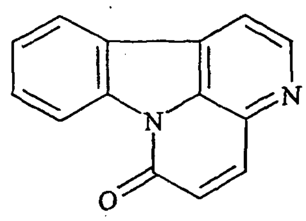

**12.** Utilisation selon l'une quelconque des revendications 1 à 11, **caractérisée en ce que** le composé de formule (I) est choisi parmi l'un des composés suivants :

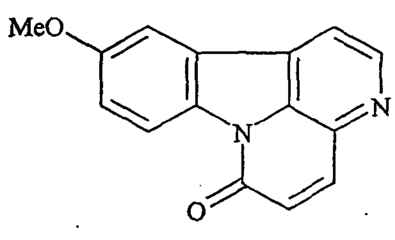

*10-méthoxy-canthin-6-one*

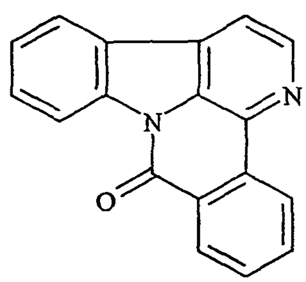

*benzo[e]canthin-6-one*

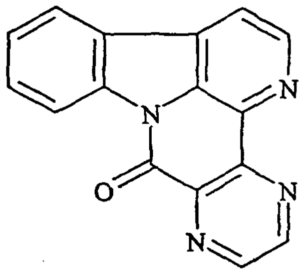

*pyrazine[e]canthin-6-one*

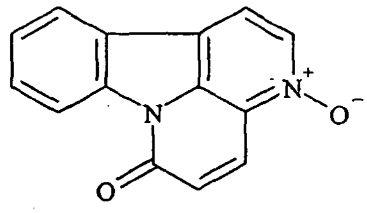

*N-oxyde-canthin-6-one*

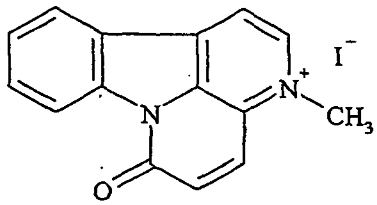

*iodure de N-méthyl-canthin-6-one*

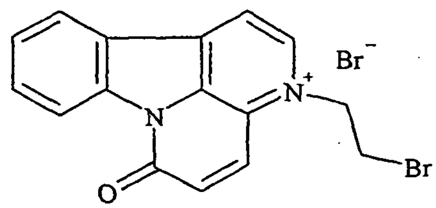

*bromure de N-bromoéthyl-canthin-6-one*

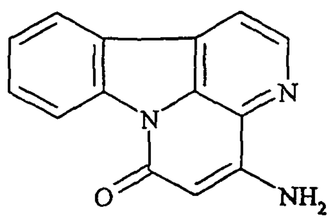

*4-amino-canthin-6-one*

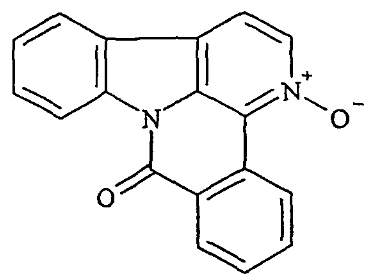

*N-oxyde-benzo[e]canthin-6-one*

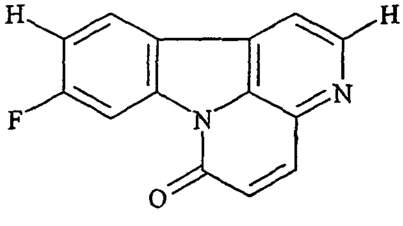

*9-fluoro -canthin-6-one*

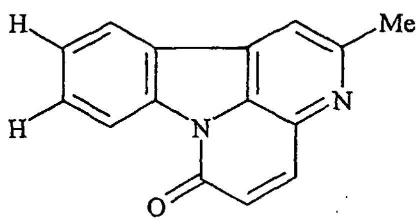

*2-méthyl-canthin-6-one*

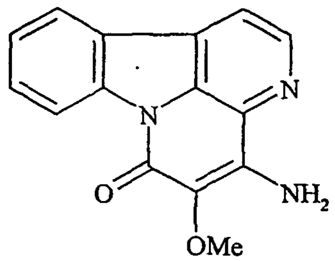

*4-amino-5-méthoxy -canthin-6-one*

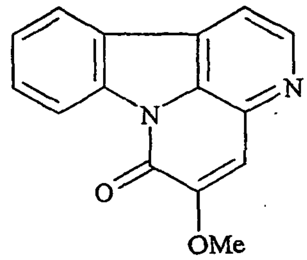

*5-méthoxy-canthin-6-one*

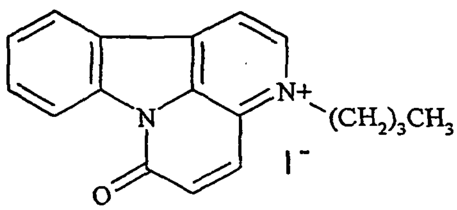

*iodure de N3-butyl-canthin-6-one*

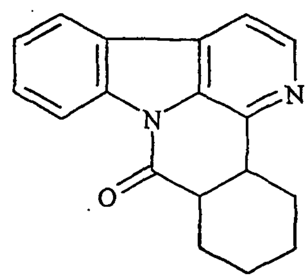

*cyclohexyl[e]-canthin-6-one*

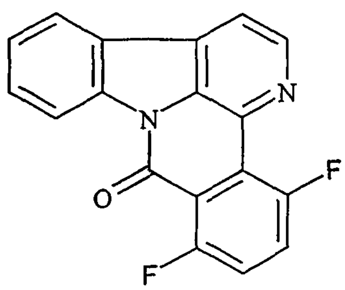

*12,15-difluoro[e]-canthin-6-one*

**13.** Utilisation selon l'une quelconque des revendications 1 à 12, **caractérisée en ce que** le composé de formule (I) est administré à une dose d'environ 0,01 mg/kg/jour à environ 100 mg/kg/jour, de préférence d'environ 0,1 à 50 mg/kg/jour, et avantageusement d'environ 1 à environ 20 mg/kg/jour.

**14.** Utilisation selon l'une quelconque des revendications 1 à 12, **caractérisée en ce que** le composé de formule (I) est administré par voie orale.

**15.** Composé répondant à la formule suivante :

*(N-oxyde-benzo[e]canthin-6-one)*

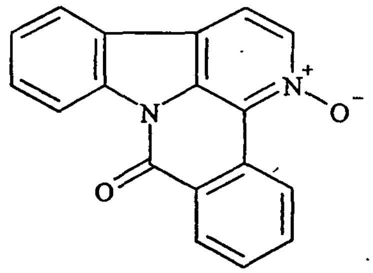

**16.** Composition pharmaceutique comprenant comme substance active le composé selon la revendication 15, en association avec un véhicule pharmaceutiquement acceptable.

## Claims

**1.** Use, in the preparation of a drug for the treatment or prevention of pathologies which are linked to, or caused by, mycobacteria, of at least one of the following Formula (I) compounds :

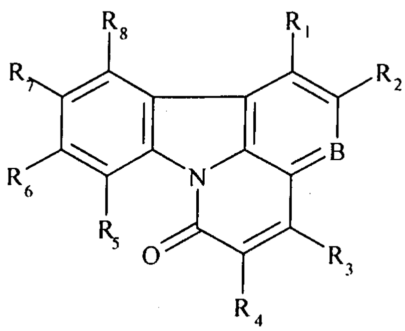

(I)

wherein :

• B represents :

* a nitrogen atom, or
* a N-oxide $N^+$-$O^-$ group, or
* a group having the formula $N^+$-R or

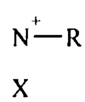

R represents an alkyl group, which may be linear, branched or cyclic, saturated or unsaturated, comprising from 1 to 12 carbon atoms, preferably from 1 to 6 carbon atoms, which may be substituted, for instance by at least one halogen atom, and
$X^-$ represents an anion which may be chosen among mineral or organic anions.

• $R_1$, $R_2$, $R_3$, $R_4$, $R_5$, $R_6$, $R_7$ and $R_8$ independently represent :

- a hydrogen atom,
- an alkyl group, which may be linear, branched or cyclic, saturated or unsaturated, comprising from 1 to 12 carbon atoms, preferably from 1 to 6 carbon atoms,
- a halogen atom, chosen among chlorine, fluorine, bromine and iodine,

- a halogenoalkyl group, wherein the alkyl chain is linear, branched or cyclic, saturated or unsaturated, comprising from 1 to 12 carbon atoms, preferably from 1 to 6 carbon atoms, and wherein the halogen atom (s) are chosen among fluorine, chlorine, bromine and iodine,
- a hydroxyl group,
- a nitro -NO group,
- a -CN group,
- a -SH group,
- a carboxylic acid -COOH group,
- an amide $-CONH_2$ group,
- an amine $-NH_2$ group,
- an alcoxy $-OR_a$ group, wherein $R_a$ represents an alkyl group, which may be linear, branched or cyclic, saturated or unsaturated, comprising from 1 to 12 carbon atoms, preferably from 1 to 6 carbon atoms,
- an alkylester $-COOR_b$ group, wherein $R_b$ represents an alkyl group, which may be linear, branched or cyclic, saturated or unsaturated, comprising from 1 to 12 carbon atoms, preferably from 1 to 6 carbon atoms,
- a secondary ($-NHCOR_c$) or tertiary ($-N(COR_d)COR_c$) alkylamide group, wherein $R_c$ and $R_d$ independently represent an alkyl group, which may be linear, branched or cyclic, saturated or unsaturated, comprising from 1 to 12 carbon atoms, preferably from 1 to 6 carbon atoms,
- a secondary ($-NHR_e$) or tertiary ($-NR_eR_f$) alkylamine group, wherein $R_e$ and $R_f$ independently represent an alkyl group, which may be linear, branched or cyclic, saturated or unsaturated, comprising from 1 to 12 carbon atoms, preferably from 1 to 6 carbon atoms,
- an alkylthio ($-SR_g$) group, wherein $R_g$ represents an alkyl group, which may be linear, branched or cyclic, saturated or unsaturated, comprising from 1 to 12 carbon atoms, preferably from 1 to 6 carbon atoms,
- a $C_2$-$C_6$ heterocyclic group, comprising from 1 to 4 heteroatoms chosen among sulphur, nitrogen and oxygen,
- a $-SO_2-NR_hR_i$ group or a $-NR_h-SO_2-R_i$ group, wherein $R_h$ and $R_i$ independently represent an alkyl group, which may be linear, branched or cyclic, saturated or unsaturated, comprising from 1 to 12 carbon atoms, preferably from 1 to 6 carbon atoms,

• the groups $R_1$, $R_2$, $R_3$, $R_4$, $R_5$, $R_6$, $R_7$ and $R_8$ may also form the following intramolecular cyclisations :

1/ cyclisation between $R^1$ and $R^2$ and/or
2/ cyclisation between $R^3$ and $R^4$ and/or
3/ cyclisation between $R^5$ and $R^6$ and/or
4/ cyclisation between $R^6$ and $R^7$ and/or
5/ cyclisation between $R^7$ and $R^8$,
6/ cyclisation between $R^3$ and B, notably when B represents $N^+$-R,
7/ cyclisation between $R^2$ and B, notably when B represents $N^+$-R,

- said thus formed cycles being notably aromatic cycles comprising from 5 to 30 carbon atoms, and which may also comprise at least one heteroatom chosen among : O, N, S, the aromatic cycles being for instance chosen among benzene, naphthalene, pyridine, pyrrole, thiophene, furan, pyrazine,
- wherein said cycles may be substituted by

- an hydrogen atom,
- an alkyl group, which may be linear, branched or cyclic, saturated or unsaturated, comprising from 1 to 12 carbon atoms, preferably from 1 to 6 carbon atoms,
- a halogen atom, which is chosen among chlorine, fluorine, bromine and iodine,
- a halogenoalkyl group, wherein the alkyl chain is linear, branched or cyclic, saturated or unsaturated, comprising from 1 to 12 carbon atoms, preferably from 1 to 6 carbon atoms, and wherein the halogen atom (s) are chosen among fluorine, chlorine, bromine and iodine,
- a hydroxyl group,
- a nitro -NO group,
- a cyano -CN group,
- a -SH group,
- a carboxylic acid -COOH group,
- an amide $-CONH_2$ group,
- an amine $-NH_2$ group,
- an alcoxy $-OR_a$ group, wherein $R_a$ represents an alkyl group, which may be linear, branched or cyclic,

saturated or unsaturated, comprising from 1 to 12 carbon atoms, preferably from 1 to 6 carbon atoms,
- an alkylester -$COOR_b$ group, wherein $R_b$ represents an alkyl group, which may be linear, branched or cyclic, saturated or unsaturated, comprising from 1 to 12 carbon atoms, preferably from 1 to 6 carbon atoms,
- a secondary (-$NHCOR_c$) or tertiary (-$N(COR_d)COR_c$) alkylamide group, wherein $R_c$ and $R_d$ independently represent an alkyl group, which may be linear, branched or cyclic, saturated or unsaturated, comprising from 1 to 12 carbon atoms, preferably from 1 to 6 carbon atoms,
- a secondary (-$NHR_c$) or tertiary (-$NR_eR_f$) alkylamine group, wherein $R_e$ and $R_f$ independently represent an alkyl group, which may be linear, branched or cyclic, saturated or unsaturated, comprising from 1 to 12 carbon atoms, preferably from 1 to 6 carbon atoms,
- an alkylthio (-SRg) group, wherein $R_g$ represents an alkyl group, which may be linear, branched or cyclic, saturated or unsaturated, comprising from 1 to 12 carbon atoms, preferably from 1 to 6 carbon atoms,
- a $C_2$-$C_6$ heterocyclic group comprising from 1 to 4 heteroatoms chosen among sulphur, nitrogen and oxygen, a -$SO_2$-$NR_hR_i$ group or a -$NR_h$-$SO_2$-$R_i$ group, wherein $R_h$ and $R_i$ independently represent an alkyl group, which may be linear, branched or cyclic, saturated or unsaturated, comprising from 1 to 12 carbon atoms, preferably from 1 to 6 carbon atoms.

**2.** Use according to Claim 1, **characterized in that** the mycobacteria are chosen among the group comprising *Mycobacterium tuberculosis, Mycobacterium bovis, Mycobacterium smegmatis, Mycobacterium africanum, Mycobacterium leprae, Mycobacterium kansasii, Mycobacterium xenopi, Mycobacterium avium intracellulaire, Mycobacterium scrofulaceum, Mycobacterium marinum, Mycobacterium fortuitum, Mycobacterium chelonei, Mycobacterium ulcerans* and *Mycobacterium abcessus,* notably *Mycobacterium tuberculosis* and *Mycobacterium bovis.*

**3.** Use according to Claim 1, **characterized in that** the pathologies which are linked to, or caused by, mycobacteria, are chosen among :

- Tuberculosis, and particularly

    - Tuberculosis of the respiratory tract (pulmonary tuberculosis, tuberculous lymphadenitis, larynx, trachea and bronchi tuberculosis, tuberculous pleurisy and tuberculous primary infection of the respiratory tract),
    - Tuberculosis of the nervous system (tuberculous meningitis, tuberculous polyneuritis, tuberculous myelitis),
    - Tuberculosis of the bone and joints,
    - Tuberculosis of the urogenital tract,
    - Peripheral tuberculous adenopathy,
    - Tuberculosis of the intestine, the peritoneum and the mesenteric glands
    - Tuberculosis of the skin and the subcutaneous cellular tissue
    - Tuberculosis of the eye and ear
    - Tuberculosis of the adrenal glands
    - Acute miliary tuberculosis

- Buruli's ulcer,
- Nosocomial infections,
- Leprosy,
- Cutaneous infections,
- Soft tissue infections,
- Osteomyelites,
- Localized abscesses,
- Lipoid pneumoniae.

**4.** Use according to any of Claims 1 to 3, **characterized in that** $X^-$ is chosen among : $Cl^-$, $Br^-$, $I^-$, $S^-$, $PO_3^-$, $NO_3^-$, acetate, oxalate, tartrate, succinate, maleate, fumarate, gluconate, citrate, malate, ascorbate, benzoate.

**5.** Use according to any of Claims 1 to 4, **characterized in that** B represents a nitrogen atom, and the compounds correspond to the following Formula (II) :

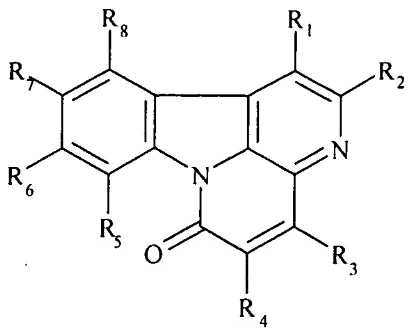

(II)

6. Use according to any of Claims 1 to 4, **characterized in that** B represents a N-oxide NO group, and the compounds correspond to the following Formula (III) :

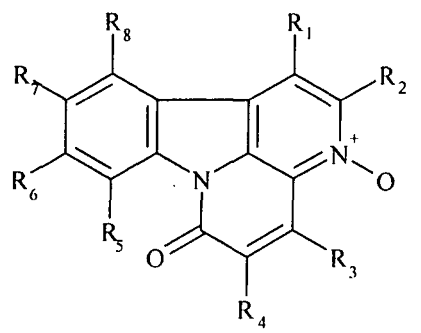

(III)

7. Use according to any of Claims 1 to 4, **characterized in that** B represents a group having Formula,

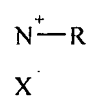

in $X^-$ and R are as defined in Claim 1, and the compounds correspond to the following Formula (IV) :

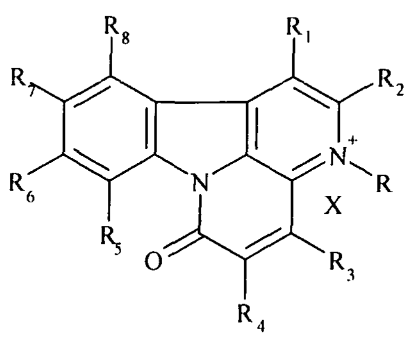

(IV)

wherein R notably represents a methyl or ethyl group, for instance substituted by at least one halogen atom such as F, Cl, Br or I.

8. Use according to any of Claims 1 to 4, **characterized in that** $R_3$ and $R_4$ form a benzene cycle between them, and the compounds correspond to the following Formula (I-1) :

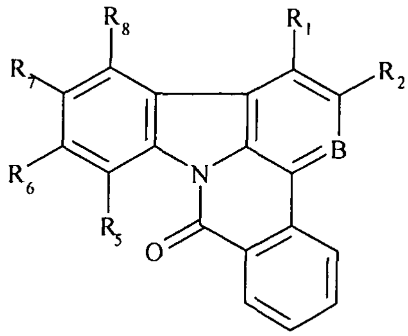

(I-1)

9. Use according to any of Claims 1 to 4, **characterized in that** the compounds are chosen among :

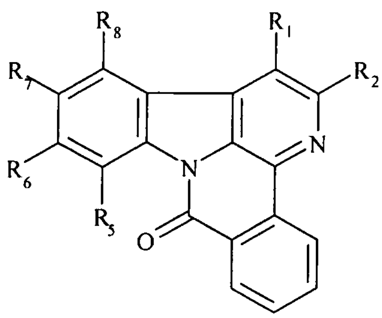

(II-1)

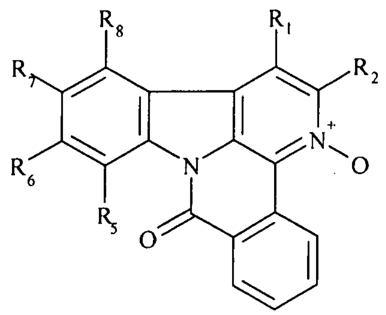

(III-1)

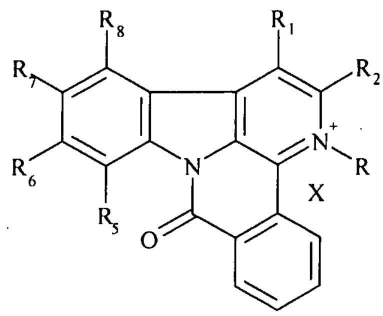

(IV-1)

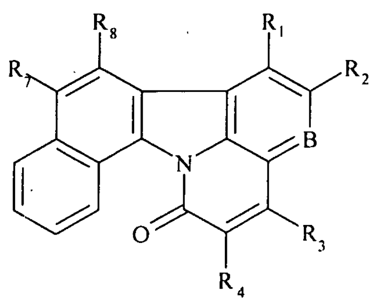

(V-1)

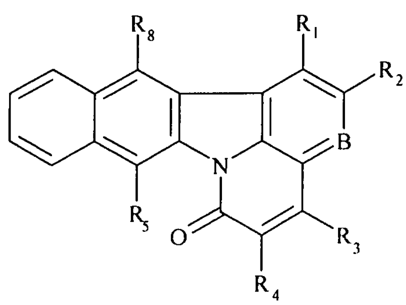

(VI-1)

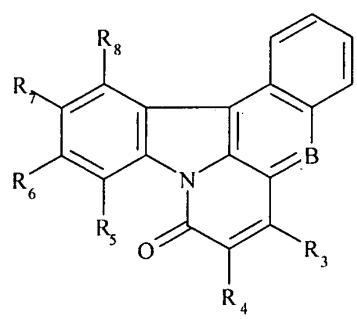

(VII-1)

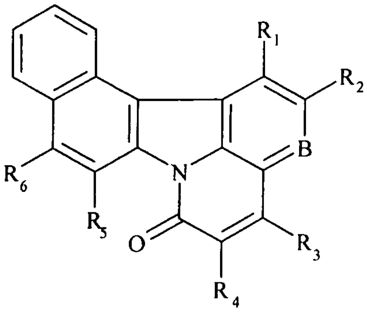

(VIII-1)

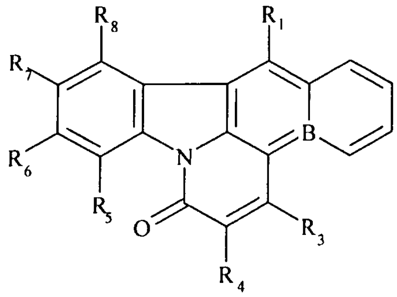

(IX-1)

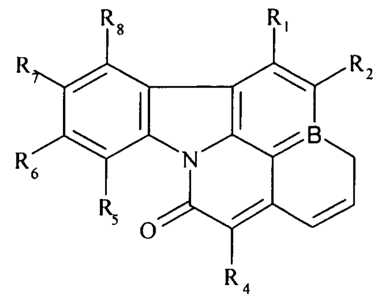

(X-1)

wherein $R_1$ - $R_8$, B and $X^-$ have the meanings as given in Claims 1 to 8.

**10.** Use according to any of from 1 to 9, **characterized in that** at least one of the radicals $R_5$ to $R_8$ and notably $R_6$ represents a halogen atom, notably a fluorine atom.

**11.** Use according to Claims 1 to 4, **characterized in that** B represents a nitrogen atom, and **in that** $R_1$, $R_2$, $R_3$, $R_4$, $R_5$, $R_6$, $R_7$ and $R_8$ represent a hydrogen atom, said compound corresponding to the following formula :

*(canthin-6-one)*

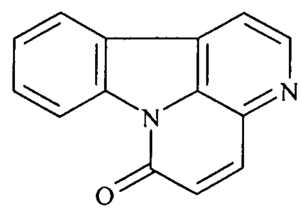

**12.** Use according to any of Claims 1 to 11, **characterized in that** the Formula (I) compound is chosen among one of the following compounds :

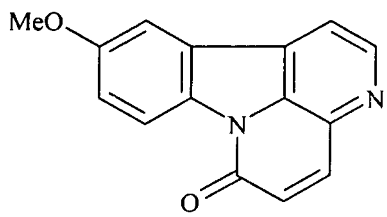

*10-methoxy-canthin-6-one*

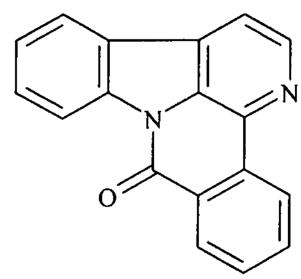

*benzo[e]canthin-6-one*

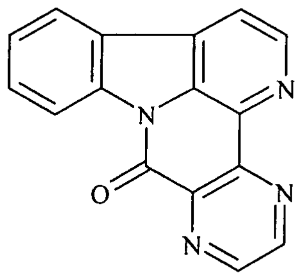

*pyrazine[e]canthin-6-one*

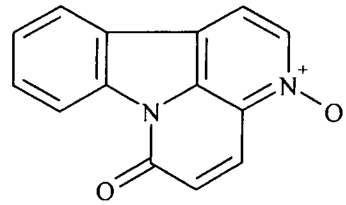

*N-oxide-canthin-6-one*

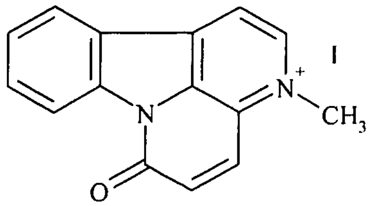

N-methyl-canthin-6-one iodide

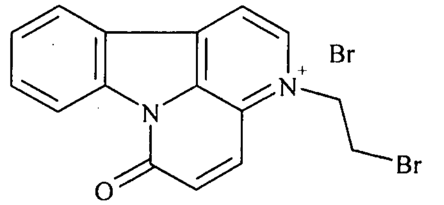

*N-bromoethyl-canthin-6-one bromide*

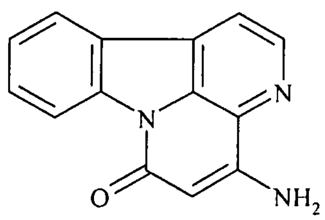

*4-amino-canthin-6-one*

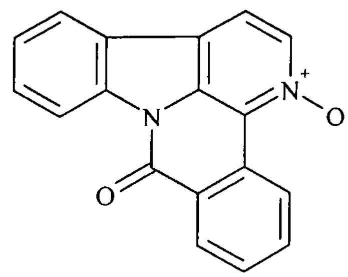

*N-oxide-benzo[e]canthin-6-one*

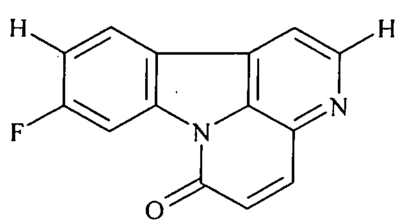

*9-fluoro -canthin-6-one*

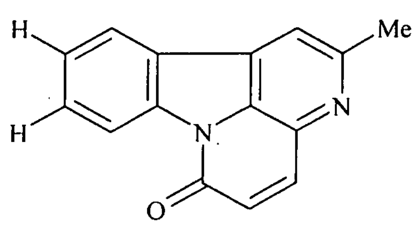

*2-methyl-canthin-6-one*

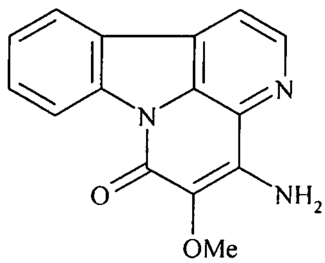

*4-amino-5-methoxy -canthin-6-one*

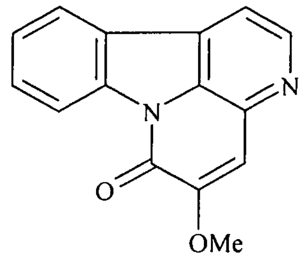

*5-methoxy-canthin-6-one*

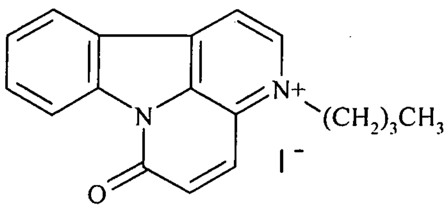

*N3-butyl-canthin-6-one iodide*

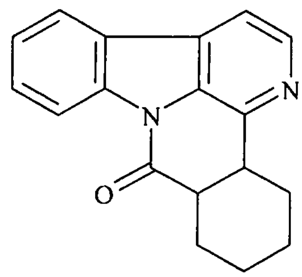

*cyclohexyl[e]-canthin-6-one*

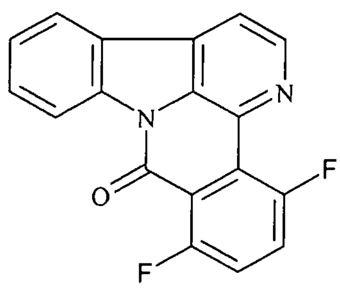

*12,15-difluoro[e]-canthin-6-one*

13. Use according to any of Claims 1 to 12, **characterized in that** the Formula (I) compound is given at a dose of from ca. 0.01 mg/kg/day to ca. 100 mg/kg/day, preferably from ca. 0.1 to 50 mg/kg/day, and advantageously from ca. 1 to ca. 20 mg/kg/day.

14. Use according to any of Claims 1 to 12, **characterized in that** the Formula (I) compound is given orally.

15. A compound corresponding to the following Formula :

*(N-oxide-benzo[e]canthin-6-one)*

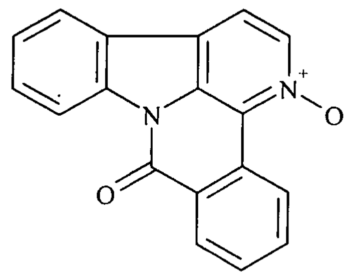

16. A pharmaceutical composition comprising as an active substance the compound according to Claim 15, in association with a pharmaceutically acceptable excipient.

**Patentansprüche**

1. Verwendung zur Herstellung eines Medikaments, das zur Behandlung oder Vorbeugung von Pathologien bestimmt ist, die mit Mykobakterien verbunden sind oder von diesen verursacht werden, mindestens einer der Verbindungen mit der folgenden Formel (I):

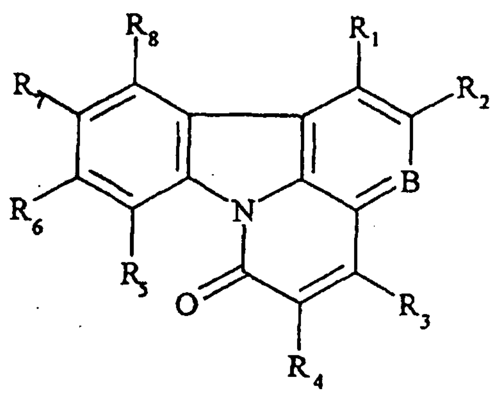

(I)

worin:

- B für:

  * ein Stickstoffatom oder
  * eine N-Oxid-Gruppe $N^+$-$O^-$ oder
  * eine Gruppe mit der Formel $N^+$-R oder

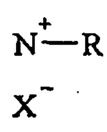

steht,

wobei R für eine gesättigte oder ungesättigte, lineare, verzweigte oder zyklische Alkylgruppe steht, die 1 bis 12 Kohlenstoffatome, bevorzugt 1 bis 6 Kohlenstoffatome umfasst, gegebenenfalls zum Beispiel mit mindestens einem Halogenatom substituiert, und $X^-$ für ein Anion steht, das aus den mineralischen oder organischen Anionen ausgewählt werden kann,

• $R_1$, $R_2$, $R_3$, $R_4$, $R_5$, $R_6$, $R_7$ und $R_8$ unabhängig voneinander für Folgendes stehen:

- ein Wasserstoffatom,
- eine gesättigte oder ungesättigte, lineare, verzweigte oder zyklische Alkylgruppe, die 1 bis 12 Kohlenstoffatome, bevorzugt 1 bis 6 Kohlenstoffatome umfasst,
- ein Halogenatom, ausgewählt aus Chlor, Fluor, Brom und Iod,
- eine Halogenalkylgruppe, worin die Alkylkette linear, verzweigt oder zyklisch, gesättigt oder ungesättigt ist, die 1 bis 12 Kohlenstoffatome, bevorzugt 1 bis 6 Kohlenstoffatome umfasst, und worin das oder die Halogenatome ausgewählt sind aus Fluor, Chlor, Brom und Iod,
- eine Hydroxylfunktion,
- eine Nitrofunktion -NO,
- eine Cyanofunktion -CN,
- eine -SH-Funktion,
- eine Carbonsäurefunktion -COOH,
- eine Amidfunktion $-CONH_2$,
- eine Aminfunktion $-NH_2$,
- eine Alkoxyfunktion $-OR_a$, wobei $R_a$ für eine gesättigte oder ungesättigte, lineare, verzweigte oder zyklische Alkylgruppe steht, die 1 bis 12 Kohlenstoffatome, bevorzugt 1 bis 6 Kohlenstoffatome umfasst,
- eine Alkylesterfunktion $-COOR_b$, wobei $R_b$ für eine gesättigte oder ungesättigte, lineare, verzweigte oder zyklische Alkylgruppe steht, die 1 bis 12 Kohlenstoffatome, bevorzugt 1 bis 6 Kohlenstoffatome umfasst,
- eine sekundäre ($-NHCOR_c$) oder tertiäre Alkylamidfunktion ($-N(COR_d)COR_c$), worin $R_c$ und $R_d$ unabhängig voneinander für eine gesättigte oder ungesättigte, lineare, verzweigte oder zyklische Alkylgruppe stehen, die 1 bis 12 Kohlenstoffatome, bevorzugt 1 bis 6 Kohlenstoffatome umfasst,
- eine sekundäre ($-NHR_e$) oder tertiäre Alkylaminfunktion ($-NR_eR_f$), worin $R_e$ und $R_f$ unabhängig voneinander für eine gesättigte oder ungesättigte, lineare, verzweigte oder zyklische Alkylgruppe stehen, die 1 bis 12 Kohlenstoffatome, bevorzugt 1 bis 6 Kohlenstoffatome umfasst,
- eine Alkylthiofunktion ($-SR_g$), wobei $R_g$ für eine gesättigte oder ungesättigte, lineare, verzweigte oder zyklische Alkylgruppe steht, die 1 bis 12 Kohlenstoffatome, bevorzugt 1 bis 6 Kohlenstoffatome umfasst,
- eine heterozyklische $C_2$-$C_6$-Gruppe, die 1 bis 4 Heteroatome, ausgewählt aus Schwefel, Stickstoff und Sauerstoff, umfasst,
- eine $-SO_2-NR_hR_i$-Gruppe oder eine $-NR_h-SO_2-R_i$-Gruppe, worin $R_h$ und $R_i$ unabhängig voneinander für eine gesättigte oder ungesättigte, lineare, verzweigte oder zyklische Alkylgruppe stehen, die 1 bis 12 Kohlenstoffatome, bevorzugt 1 bis 6 Kohlenstoffatome umfasst,

• wobei die Gruppen $R_1$, $R_2$, $R_3$, $R_4$, $R_5$, $R_6$, $R_7$ und $R_8$ auch die folgenden intramolekularen Cyclisierungen bilden können:

1/ Cyclisierung zwischen $R^1$ und $R^2$ und/oder
2/ Cyclisierung zwischen $R^3$ und $R^4$ und/oder
3/ Cyclisierung zwischen $R^5$ und $R^6$ und/oder
4/ Cyclisierung zwischen $R^6$ und $R^7$ und/oder
5/ Cyclisierung zwischen $R^7$ und $R^8$,
6/ Cyclisierung zwischen $R^3$ und B, insbesondere wenn B für $N^+$-R steht,
7/ Cyclisierung zwischen $R^2$ und B, insbesondere wenn B für $N^+$-R steht,

- wobei die so gebildeten Ringe insbesondere aromatische Ringe sind, die 5 bis 30 Kohlenstoffatome umfassen, die gegebenenfalls mindestens ein Heteroatom umfassen, ausgewählt aus: O, N, S, wobei die aromatischen Ringe zum Beispiel aus Benzol, Naphtalen, Pyridin, Pyrrol, Thiophen, Furan, Pyrazin ausgewählt sind,
- wobei die Ringe gegebenenfalls substituiert sind mit
- einem Wasserstoffatom,
- einer gesättigten oder ungesättigten, linearen, verzweigten oder zyklischen Alkylgruppe, die 1 bis 12 Kohlenstoffatome, bevorzugt 1 bis 6 Kohlenstoffatome umfasst,
- einem Halogenatom, ausgewählt aus Chlor, Fluor, Brom und Iod,
- einer Halogenalkylgruppe, worin die Alkylkette linear, verzweigt oder zyklisch, gesättigt oder ungesättigt ist, die 1 bis 12 Kohlenstoffatome, bevorzugt 1 bis 6 Kohlenstoffatome umfasst, und worin das oder die Halogenatome ausgewählt sind aus Fluor, Chlor, Brom und Iod,
- einer Hydroxylfunktion,

- einer Nitrofunktion -NO,
- einer Cyanofunktion -CN,
- einer -SH-Funktion,
- einer Carbonsäurefunktion -COOH,
- einer Amidfunktion $-CONH_2$,
- einer Aminfunktion $-NH_2$,
- einer Alkoxyfunktion $-OR_a$, wobei $R_a$ für eine gesättigte oder ungesättigte, lineare, verzweigte oder zyklische Alkylgruppe steht, die 1 bis 12 Kohlenstoffatome, bevorzugt 1 bis 6 Kohlenstoffatome umfasst,
- einer Alkylesterfunktion $-COOR_b$, wobei $R_b$ für eine gesättigte oder ungesättigte, lineare, verzweigte oder zyklische Alkylgruppe steht, die 1 bis 12 Kohlenstoffatome, bevorzugt 1 bis 6 Kohlenstoffatome umfasst,
- einer sekundären ($-NHCOR_c$) oder tertiären Alkylamidfunktion ($-N(COR_d)COR_c$), worin $R_c$ und $R_d$ unabhängig voneinander für eine gesättigte oder ungesättigte, lineare, verzweigte oder zyklische Alkylgruppe stehen, die 1 bis 12 Kohlenstoffatome, bevorzugt 1 bis 6 Kohlenstoffatome umfasst,
- einer sekundären ($-NHR_e$) oder tertiären Alkylaminfunktion ($-NR_eR_f$), worin $R_e$ und $R_f$ unabhängig voneinander für eine gesättigte oder ungesättigte, lineare, verzweigte oder zyklische Alkylgruppe stehen, die 1 bis 12 Kohlenstoffatome, bevorzugt 1 bis 6 Kohlenstoffatome umfasst,
- einer Alkylthiofunktion ($-SR_g$), wobei $R_g$ für eine gesättigte oder ungesättigte, lineare, verzweigte oder zyklische Alkylgruppe steht, die 1 bis 12 Kohlenstoffatome, bevorzugt 1 bis 6 Kohlenstoffatome umfasst,
- einer heterozyklischen $C_2$-$C_6$-Gruppe, die 1 bis 4 Heteroatome, ausgewählt aus Schwefel, Stickstoff und Sauerstoff, umfasst,
- einer $-SO_2-NR_hR_i$-Gruppe oder einer $-NR_h-SO_2-R_i$-Gruppe, worin $R_h$ und $R_i$ unabhängig voneinander für eine gesättigte oder ungesättigte, lineare, verzweigte oder zyklische Alkylgruppe stehen, die 1 bis 12 Kohlenstoffatome, bevorzugt 1 bis 6 Kohlenstoffatome umfasst,

**2.** Verwendung nach Anspruch 1, **dadurch gekennzeichnet, dass** die Mykobakterien ausgewählt sind aus der Gruppe gebildet aus *Mycobacterium tuberculosis, Mycobacterium bovis, Mycobacterium smegmatis, Mycobacterium africanum, Mycobacterium leprae, Mycobacterium kansasii, Mycobacterium xenopi, Mycobacterium avium intracellulaire, Mycobacterium scrofulaceum, Mycobacterium marinum, Mycobacterium fortuitum, Mycobacterium chelonei, Mycobacterium ulcerans* et *Mycobacterium abcessus,* insbesondere *Mycobacterium tuberculosis* und *Mycobacterium bovis.*

**3.** Verwendung nach Anspruch 1, **dadurch gekennzeichnet, dass** die Pathologien, die mit Mykobakterien verbunden sind oder von diesen verursacht werden, ausgewählt sind aus:

- Tuberkulose und vor allem

  - Tuberkulose der Atmungsorgane (Tuberkulose der Lungen, der Brustganglien, des Kehlkopfs, der Luftröhre, der Bronchien, Pleuritis tuberculosa und tuberkulöser Erstinfektion der Atmungsorgane),
  - Tuberkulose des Nervensystems (tuberkulöser Meningitis, tuberkulöser Polyneuritis, tuberkulöser Myelitis),
  - Tuberkulose der Knochen und Gelenke,
  - Tuberkulose des Urogenitalapparats,
  - tuberkulöser Adenopathie,
  - Tuberkulose des Darms, des Bauchfells und der mesenterischen Ganglien,
  - Tuberkulose der Haut und des Unterhautzellgewebes,
  - Tuberkulose des Auges und des Ohres,
  - Tuberkulose der Nebennieren,
  - Miliartuberkulose,

- Buruli-Ulkus,
- nosokomialen Infektionen,
- Lepra,
- Hautinfektionen,
- Infektionen der Weichgewebe,
- Knochenmarkentzündungen,
- lokalen Abszessen,
- lipoiden Pneumonien.

**4.** Verwendung nach irgendeinem der Ansprüche 1 bis 3, **dadurch gekennzeichnet, dass** $X^-$ ausgewählt ist aus: $Cl^-$, $Br^-$, $I^-$, $S^-$, $PO_3^-$, $NO_3^-$, Acetat, Oxalat, Tartrat, Succinat, Maleat, Fumarat, Gluconat, Citrat, Malat, Ascorbat, Benzoat.

**5.** Verwendung nach irgendeinem der Ansprüche 1 bis 4, **dadurch gekennzeichnet, dass** B für ein Stickstoffatom steht, wobei die Verbindungen der folgenden Formel (II) entsprechen:

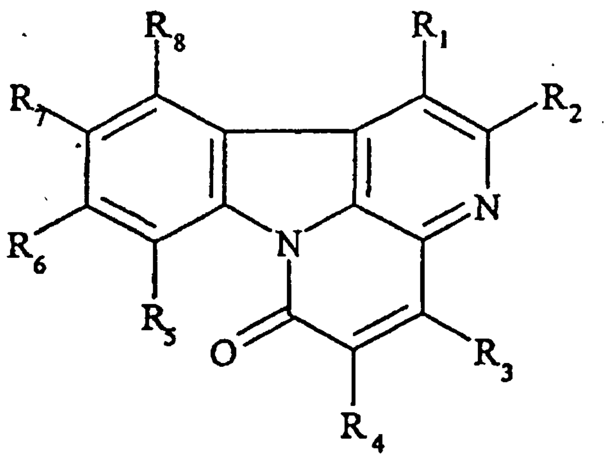

(II)

**6.** Verwendung nach irgendeinem der Ansprüche 1 bis 4, **dadurch gekennzeichnet, dass** B für eine N-Oxid-Gruppe NO steht, wobei die Verbindungen der folgenden Formel (III) entsprechen:

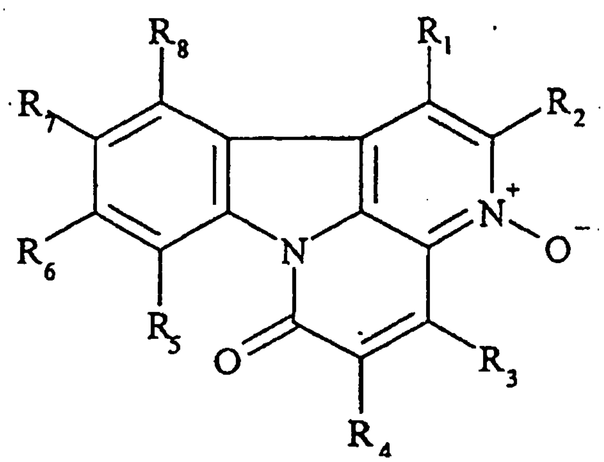

(III)

**7.** Verwendung nach irgendeinem der Ansprüche 1 bis 4, **dadurch gekennzeichnet, dass** B für eine Gruppe mit der Formel

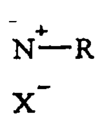

steht, wobei $X^-$ und R so sind, wie in Anspruch 1 definiert, wobei die Verbindungen der folgenden Formel (IV) entsprechen:

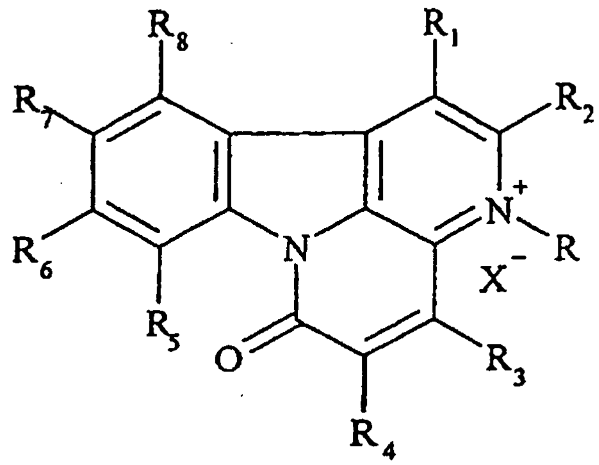

(IV)

wobei R insbesondere für eine Methyl-, Ethylgruppe steht, die zum Beispiel mit mindestens einem Halogenatom, wie etwa F, Cl, Br oder I, substituiert ist.

8. Verwendung nach irgendeinem der Ansprüche 1 bis 4, **dadurch gekennzeichnet, dass** $R_3$ und $R_4$ miteinander einen Benzolring bilden, wobei die Verbindungen der folgenden Formel (I-1) entsprechen:

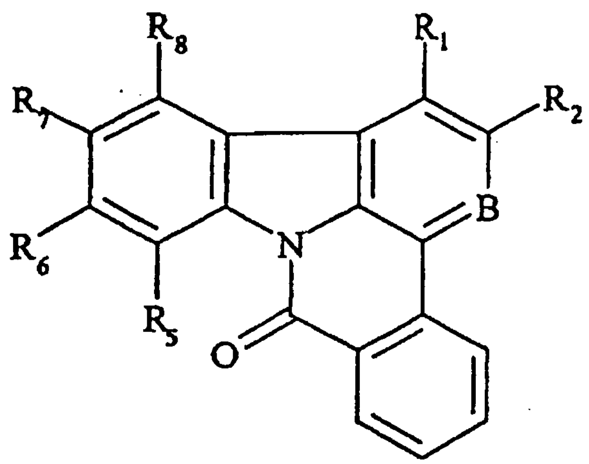

(I-1)

9. Verwendung nach irgendeinem der Ansprüche 1 bis 4, **dadurch gekennzeichnet, dass** die Verbindungen ausgewählt sind aus:

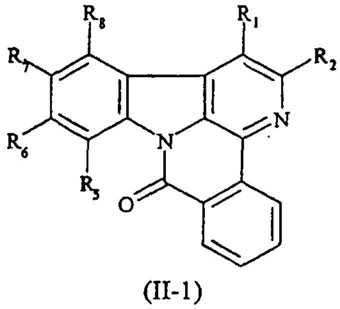

(II-1)

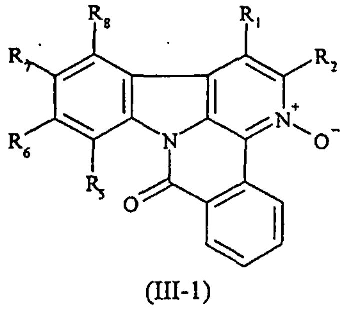

(III-1)

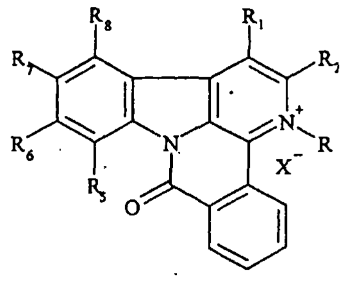

(IV-1)

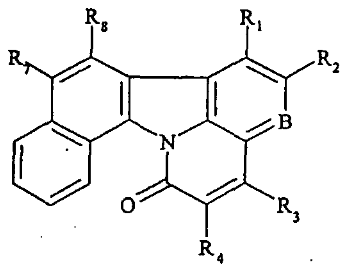

(V-1)

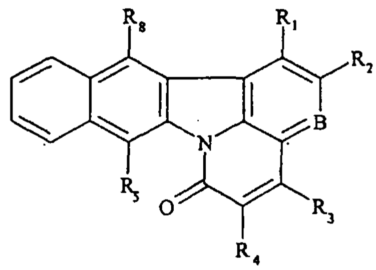

(VI-1)

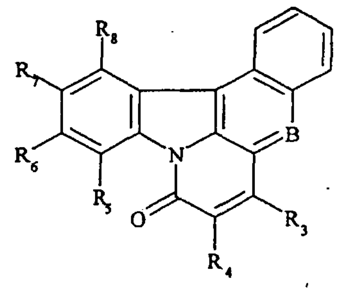

(VII-1)

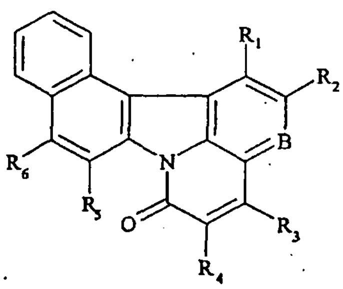

(VIII-1)

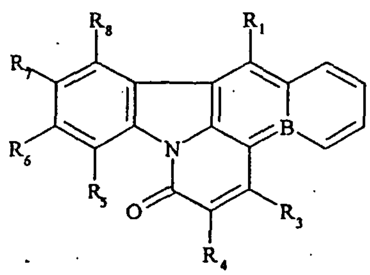

(IX-1)

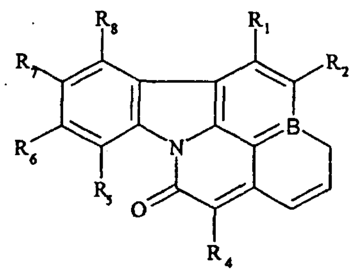

(X-1)

worin $R_1$ bis $R_8$, B und $X^-$ die Bedeutungen haben, die in den Ansprüchen 1 bis 8 angegeben sind.

**10.** Verwendung nach irgendeinem der Ansprüche 1 bis 9, **dadurch gekennzeichnet, dass** mindestens einer der Reste $R_5$ bis $R_8$ und insbesondere $R_6$ für ein Halogenatom, insbesondere ein Fluoratom steht.

**11.** Verwendung nach Anspruch 1 bis 4, **dadurch gekennzeichnet, dass** B für ein Stickstoffatom steht und dadurch, dass $R_1$, $R_2$, $R_3$, $R_4$, $R_5$, $R_6$, $R_7$ und $R_8$ für ein Wasserstoffatom stehen, wobei die Verbindung der folgenden Formel entspricht:

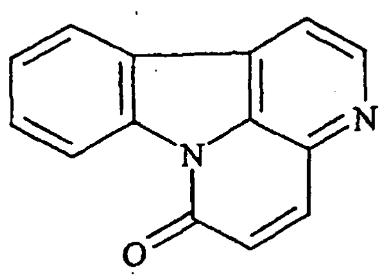

(Canthin-6-on)

**12.** Verwendung nach irgendeinem der Ansprüche 1 bis 11, **dadurch gekennzeichnet, dass** die Verbindung mit der Formel (I) aus einer der folgenden Verbindungen ausgewählt ist:

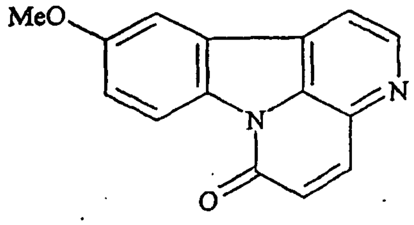

*10-Methoxy-canthin-6-on*

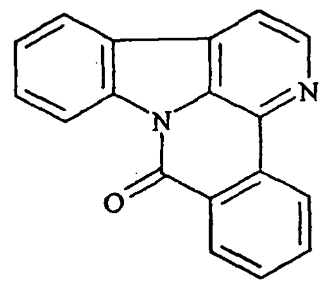

*Benzo[e]canthin-6-on*

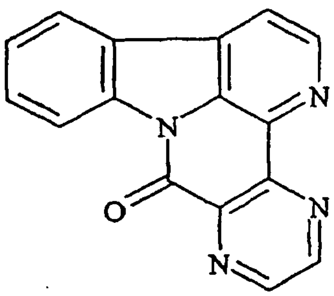

*Pyrazin[e]canthin-6-on*

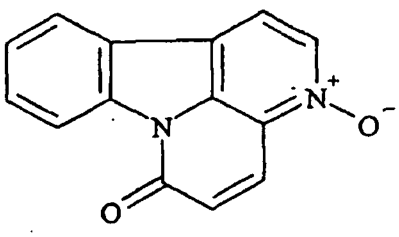

*N-Oxid-canthin-6-on*

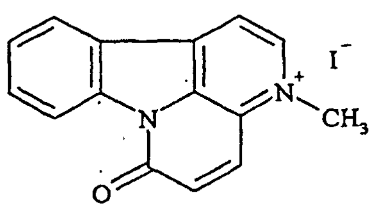

*N-Methyl-canthin-6-on-iodid*

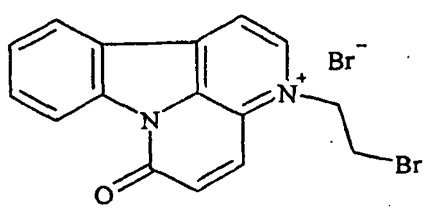

*N-Bromethyl-canthin-6-on-bromid*

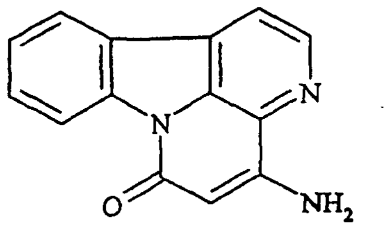

4-Amino-canthin-6-on

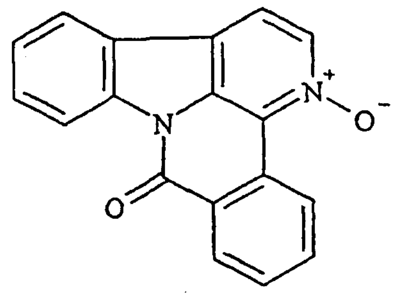

N-Oxid-benzo[e]canthin-6-on

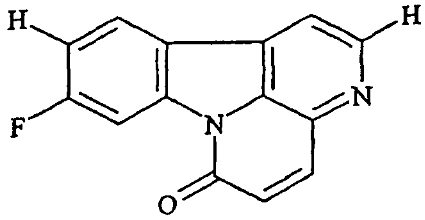

9-Fluor-canthin-6-on

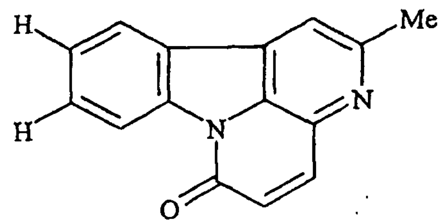

2-Methyl-canthin-6-on

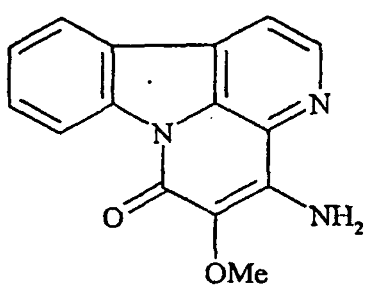

4-Amino-5-methoxy-canthin-6-on

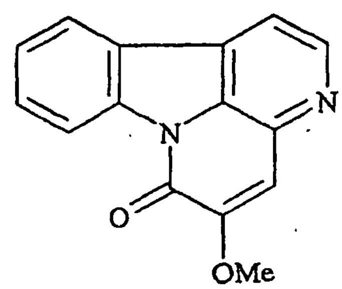

5-Methoxy-canthin-6-on

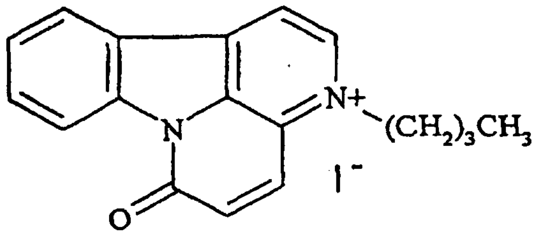

*N3-Butyl-canthin-6-on-iodid*

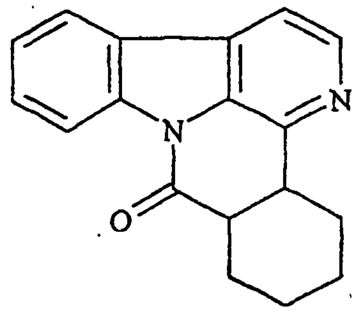

*Cyclohexyl[e]-canthin-6-on*

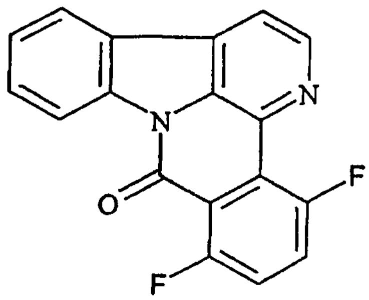

*12,15-Difluor[e]-canthin-6-on*

**13.** Verwendung nach irgendeinem der Ansprüche 1 bis 12, **dadurch gekennzeichnet, dass** die Verbindung mit der Formel (I) mit einer Dosis von etwa 0,01 mg/kg/Tag bis etwa 100 mg/kg/Tag, bevorzugt etwa 0,1 bis 50 mg/kg/Tag, und vorteilhafterweise mit etwa 20 mg/kg/Tag verabreicht wird.

**14.** Verwendung nach irgendeinem der Ansprüche 1 bis 12, **dadurch gekennzeichnet, dass** die Verbindung mit der Formel (I) oral verabreicht wird:

**15.** Verbindung, die der folgenden Formel entspricht:

*(N-Oxid-benzo[e]-canthin-6-on)*

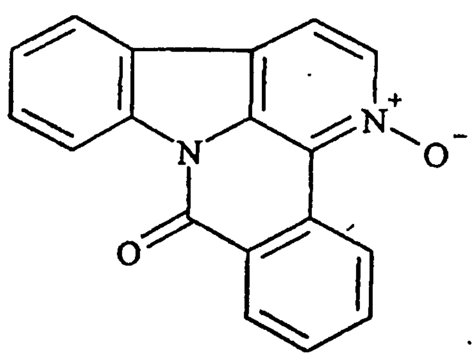

**16.** Pharmazeutische Zusammensetzung, die als Wirkstoff die Verbindung nach Anspruch 15 in Assoziation mit einem pharmazeutisch verträglichen Vehikel umfasst.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2004050092 A


### Littérature non-brevet citée dans la description


- **Ohmoto et al.** *Chem. Pharm. Bull.,* 1976, vol. 24, 1532-1536
- **Fukamiya et al.** *Planta Med.,* 1987, vol. 53, 140-143
- **Kachanapoom et al.** *Phytochemistry,* 2001, vol. 56, 383-386
- **Ma et al.** *Phytochemistry,* 2000, vol. 53, 1075-1078
- **Timo Ulrichs et al.** Modified immunohistological staining allows detection of Ziehl-Neelsen-negativé Mycobacterium tuberculosis organisms and their precise localization in human tissue. *J. Pathol.,* 2005, vol. 205, 633-640
- **Soriano-Agatón et al.** *Journal of Natural Products,* Novembre 2005, vol. 68 (11